# EUROPEAN PATENT APPLICATION

(11) **EP 1 995 323 A1**
(43) Date of publication of application: **26.11.2008**
(21) Application number: 07108889.2
(22) Date of filing: 24.05.2007
(51) Int. Cl.: C12P 17/00, C12P 19/02, C12P 19/26, C12P 19/28, C07D 309/10, C07D 309/14, C07D 335/02, C07H 1/02, C07H 5/10, C07H 11/04, C07H 15/04, C07H 15/12, C07D 211/40, C07D 211/54, C07D 211/56, C07D 309/08

(54) **Method for preparing C-6 phosphorylated D-aldohexoses and C-6 phosphorylated D-aldohexose derivatives**

(71) Applicant: Libragen, 31000 Toulouse (FR)
(72) Inventor: Auriol, Daniel, 31300, Toulouse (FR); Nalin, Renaud, 31280, Dremil-Lafage (FR); Lefevre, Fabrice, 32120, Bajonnette (FR); Ginolhac, Aurélien, 31000, Toulouse (FR); de Guembecker, Daphné, 31000, Toulouse (FR); Zago, Caroline, 31400, Toulouse (FR)
(74) Representative: Tezier Herman, Béatrice

(57) **Abstract**

The present invention concerns a method for preparing C-6 phosphorylated D-aldohexoses and C-6 phosphorylated D-aldohexose derivatives, and uses thereof. The method comprises incubating polyphosphate as sole source of phosphate donor and a polyphosphate-glucose phosphotransferase (polyphosphate: D-glucose 6-phosphotransferase, EC 2.7.1.63) in an aqueous solution with a D-aldohexose or a D-aldohexose derivative.

## Description

### FIELD OF THE INVENTION

The present invention concerns a method for preparing C-6 phosphorylated D-aldohexoses and C-6 phosphorylated D-aldohexose derivatives, new C-6 phosphorylated D-aldohexoses derivatives and uses thereof.

### BACKGROUND OF THE INVENTION

Phosphorylated sugars are important structural and energy intermediates in the living species, from prokaryotes to higher eukaryotes. Several aldohexose derivatives occur in nature under phosphorylated form. The two most common phosphorylation positions are R1=OPO₃H₂ and the C-6 carbon where the primary alcohol group is replaced by OPO₃H₂. As an example amongst phosphorylated aldohexoses, glucose-6-phosphate is a key glucose intermediate used in cells at the cross road of several metabolism pathways: glycogen synthesis (glyconeogenesis), fatty acid synthesis, amino acid synthesis from pyruvic acid and nucleic acid synthesis from pentoses.
Phosphorylated aldohexoses derivatives are interesting compounds either for their own properties (addition of an anionic part to a highly polar neutral molecule), or as chemical intermediates for other synthesis.
Specifically, glucosamine-6-phosphate is synthesized from fructose-6-phosphate and glutamine (Ghosh S, Blumenthal HJ, Davidson E, Roseman S. Glucosamine metabolism. V. Enzymatic synthesis of glucosamine 6-phosphate, J Biol Chem, 1960 235: 1265 - 1273) as the first step of the hexosamine biosynthesis pathway. The end-product of this pathway is UDP-N-acetylglucosamine, which is then used for making proteoglycans, glycolipids and glycosaminoglycans that are major components of join cartilage. Therefore glucosamine-6-phosphate is useful as a nutritional supplement for osteoarthritis and knee pain treatment.
The phosphorylated derivative of glucosamine at the primary alcohol group, glucosamine-6-phosphate was shown to have an allosteric regulation effect of glycogen synthase and hexokinase during glucosamine-induced insulin resistance in skeletal muscle and heart (Virkamaki A, Yki-Jarvinen H. Allosteric regulation of glycogen synthase and hexokinase by glucosamine-6-phosphate during glucosamine-induced insulin resistance in skeletal muscle and heart, Diabetes, 1999 48 (5): 1101 - 1107.)
Wilson and Chung (Wilson JE, Chung V. Rat brain hexokinase: further studies on the specificity of the hexose and hexose 6-phosphate binding sites, Arch Biochem Biophys, 1989 269 (2): 517 - 525) have shown that 5-thioglucose phosphorylated derivatives are of interest to inhibit kinases. They demonstrated that the inhibition constant Ki of rat brain hexokinase in the case of 5-thioglucose 6-phosphate is 10-fold lower than that seen with glucose 6-phosphate.
More generally, due to their key position in living species or their structural similarity, these compounds are good building blocks to create new drugs (such as kinase inhibitors), nutritional supplements for animals and humans, cosmetic ingredients (moisturizers), detergents or new polymers. However, production of aldohexose derivatives phosphorylated at the primary alcohol group with industrial compatible specifications (such as cost, yield, and quantities) remains a key issue.
Chemical phosphorylation of hydroxylated compounds can be achieved by using activated phosphorus derivatives such as POCl₃, PCl₃, PSCl₃ and their diethyl esters. These activated phosphorus derivatives are intermediates in the production of organophosphorous pesticides, plastics, flame retardants and hydraulic fluids; though they are produced at very large quantities (more than 500 000 000 pounds per year in US), they are classified as highly toxic to mammals based on acute oral and inhalation data with rats (Segall Y, Quistad GB, Sparks SE, Casida JE. Major intermediates in organophosphate synthesis (PCl3, POCl3, PSCl3, and their diethyl esters) are anticholinesterase agents directly or on activation, Chem Res Toxicol, 2003 16 (3): 350 - 356). In addition, these activated phosphorus intermediates are very poorly stable in aqueous media and anhydrous solvents must be used. As far as carbohydrates are concerned, several works report the phosphorylation of cellulose with POCl₃ (in particular: Zeronian SH, Adams S, Alger K, Lipska AE. Phosphorylation of cellulose: effect of the reactivity of the starting polymer on the properties of the phosphorylated product, J Appl Polym Sci, 1980 25 (3): 519 - 528); US 6,479,656 describes the production of α-methyl-6-O-acetyl-D-mannopyranoside-2,3,4-trisphosphate by reacting the expensive activated phosphorus derivative dibenzyl N,N'-diethylphosphoramidite with trimethylchlorosilane and α-methyl-6-O-acetyl-D-mannopyranoside in dry tetrahydrofuran. No data is given regarding the relative reactivity of the hydroxy groups at C-2, C-3 and C-4. Nevertheless, it is observed that when the stoichiometry is sufficient, all the secondary alcohol groups can be substituted in one hand and in the other hand, it can be stated that the primary alcohol group should also have reacted if it has not been substituted by the acetyl group. Such a lack of regioselectivity is not observed when using enzymes as catalysts and this is an advantage of the process of the present invention; when a specific alcohol group of a carbohydrate (aldohexoses contain 5 alcohol groups taking into account the one which is formed during cyclization) has to be modified, it has to be found generally a chemical procedure that is able to protect and then deprotect the alcohol groups that must remain unsubstituted.
Patent JP61140595 describes a chemical process for the synthesis of a sugar phosphate without any preliminary chemical protection to the sugar skeleton. The compound is prepared by reaction of a specific phosphoric acid monoester with glycidol (also called (R)-3-hydroxy-1,2-epoxypropane). More specifically, the synthesis is achieved by reacting 1mol of a phosphoric acid monoester (salt) (e.g. glucose-1-phosphate) with preferably 1-5mol of glycidol in the presence of an inert solvent (preferably water) at 40-90 °C. However this reference does not describe or teach any chemical process that would enable the chemical phosphorylation of aldohexoses and of their derivatives without any preliminary protection steps.
In living organisms, phosphorylation of aldohexoses is achieved by kinases (EC 2.7.1: phosphotransferases with an alcohol group as acceptor) that are substrate specific, and that mainly use ATP as a source of phosphate, thus releasing ADP. Glucose-6-phosphate is synthesized in cells from glucose and ATP with a glucokinase (EC 2.7.1.2). The main drawback of this approach is the cost of ATP depending on the cost of the final product. Thus, a process of production of D-glucose-6-phosphate with simultaneous ATP regeneration has been developed up to the commercialisation scale (Ishikawa H, Takase S, Tanaka T, Hikita H. Experimental investigation of G6P production and simultaneous ATP regeneration by conjugated enzymes in an ultrafiltration hollow-fiber reactor, Biotechnol Bioeng, 1989 34 (3): 369 - 379). Glucose-6-phosphate synthesis is also proposed by Berke et al. using a high molecular weight derivative of ATP in an enzyme membrane reactor (Berke W, Schüz HJ, Wandrey C, Morr M, Denda G, Kula MR. Continuous regeneration of ATP in enzyme membrane reactor for enzymatic syntheses, Biotechnol Bioeng, 1988 32(2): 130 - 139). Some other transferases are able to transfer a carbohydrate from a donor substrate to inorganic phosphate as acceptor to form a glycoside with phosphate as aglycone. An example of this kind of enzyme is sucrose phosphorylase (EC 2.4.1.7) that catalyses the synthesis of α-D-glucose-1-phosphate from sucrose, the donor substrate, and orthophosphate (Guibert A, Monsan P. Production and purification of sucrose phosphorylase from Leuconostoc mesenteroides, Ann NY Acad Sci, 1988 542: 307 - 311). Another example of this group of hexosyltransferases is provided by JP9224691 wherein it is described an enzymatic process for the production of two sugar phosphate: α-D-glucosamine-1-phosphate and α-D-galactose-1-phosphate. These sugar phosphates are obtained by reacting chitobiose or lactose with inorganic phosphate in the presence of cellobiose phosphorylase (EC 2.4.1.20) derived from Cellvibrio gilvus (ATCC 13127) in an amount of 0.01-100 unit/ml based on 1mM of the chitobiose or lactose at 37°C for 32 hr. For all transferases using saccharides as glycosyl donor and inorganic phosphate as acceptor, it is only obtained monosaccharide-1-phosphate; such type of enzyme is not convenient to obtain aldohexose phosphorylated derivatives at a position different from the anomeric carbon.
Some kinases have been identified as using another source of phosphate than ATP. Among these enzymes, polyphosphate sugar-kinases utilize polyphosphate polymers, a cheap source of phosphate, to phosphorylate sugars. More specifically, D-glucose-6-phosphotransferase also called polyphosphate-glucose 6-phosphotransferase (PGPTase; EC 2.7.1.63) or polyphosphate glucokinase (PPGK) catalyzes the synthesis of D-glucose-6-phosphate using long chain polyphosphates as phosphate donors and glucose as phosphate acceptor:

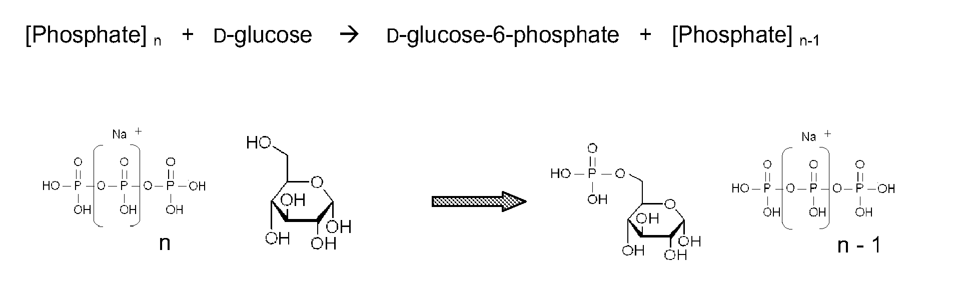

This polyphosphate-glucose phosphotransferase enzyme is particularly advantageous in that it is able to use inorganic polyphosphates (phosphate glass) which are rather cheap and abundant substances.
Tables below reports data gathered on polyphosphate-glucose phosphotransferase enzymatic activity characteristic to some groups of bacteria including Actinomycetes, in particular mycobacteria, and other phylogenetically related species (Table 1 reports data dealing with Mycobacteria and Table 2 reports data dealing with other microorganisms (source: www.brenda.uni-koeln.de/php/result flatphp4?ecno=2.7.1.63&organism)).

**Table 1: Mycobacteria described to produce polyphosphate-glucose phosphotransferase enzymatic activity**

| Microorganism | Phosphate donor | Phosphate acceptor | Comments |
|---|---|---|---|
| *M. aurum* | Polyphosphate | D-glucose | Girbal et al. Enzyme Microb Technol, 1989 11: 518 - 527 |
| *M. bovis* | Polyphosphate | D-glucose | 265 amino acids; mol. weight: 24147 M. gordonae Polyphosphate D-glucose (sequence Q7TY27) |
| M. gordonae | Polyphosphate | D-glucose | Girbal et al. Enzyme Microb Technol, 1989 11: 518 - 527 |
| M. jucho | Polyphosphate | D-glucose | Szymona O, Szymona M. Acta Microbiol Pol, 1978 27: 73 - 76 |
| M. leprae | Polyphosphate | D-glucose | M. jucho Polyphosphate D-glucose 324 amino acids; mol. weight: 34102 (sequence Q49988) |
| M. pellegrino | Polyphosphate | D-glucose | Szymona O, Szymona M. Acta Microbiol Pol, 1978 27: 73-76 |
| | | | M. pellegrino Polyphosphate D-glucose Glucose inhibits above 150 mM. |
| | | | Poly(P) inhibition above 0.05 mM. |
| | | | Km (glucose): 0,28 / 0,48 mM (37/30°C). |
| | | | Km (P of PolyP): 0.175 mM (pH 8.5; 37°C). |
| M. phlei | Polyphosphate | D-glucose | Km (PolyP15): 0.0072 mM (pH 8.5; 30°C). |
| | | | Specific activity: 140 U/mg. |
| | | | pH opt.: 8.5 (or 6.4) |
| | | | Molecular weight: 113 or 275 kDa. |
| | | | Monomer: 34 kDa |
| | | | M. smegmatis Polyphosphate D-glucose Isoelectric point: 5.0 (± 0.2). |
| M. smegmatis | Polyphosphate | D-glucose | Girbal et al. Enzyme Microb Technol, 1989 11: 518 - 527 |
| Mycobacterium sp. | Polyphosphate | D-glucose | Szymona O, Szymona M. Acta Microbiol Pol, 1978 27: 73 - 76 |
| Mycobacterium sp. JLS | Polyphosphate | D-glucose | 269 amino acids; mol. weight: 27856 (sequence Q1U3L2) |
| Mycobacterium sp. KMS | Polyphosphate | D-glucose | 269 amino acids; mol. weight: 27870 (sequence Q1TE91) |
| Mycobacterium sp. MCS | Polyphosphate | D-glucose | 269 amino acids; mol. weight: 27870 (sequence available) |
| M. thermoresistibile | Polyphosphate | D-glucose | Girbal et al. Enzyme Microb Technol, 1989 11: 518-527 |
| M. tuberculosis | | | Fru6P and Glc6P inhibit. |
| | | | Km (glucose): 0.06 mM (pH 7.5) and 0.22 mM (pH 8.6). |
| | Polyphosphate | D-glucose | Km (PolyP): 0.033 mM (pH 7.5) and 0.082 mM (pH 8.5). |
| | | | Turnover (PP35): 196 s⁻¹. |
| | | | Specific activity: 203 U/mg. |
| | | | 265 amino acids; mol. weight: 27429 (sequence Q59568) |
| | | | ADP and AMP inhibit. |
| | | | Km (ATP): 0.88 mM (pH 7.5) and 1.4 mM (pH 8.6). |
| | ATP | D-glucose | ATP inhibits at high concentration (Ki: 6.6/7.4 mM (pH 7.5/pH 8.6). |
| | | | Fru6P and D-xylose inhibit. |
| | | | Turnover: 116 s⁻¹. |
| | CTP | D-glucose | |
| Mycobacterium vanbaalenii PYR-1 | Polyphosphate | D-glucose | 272 amino acids; mol. weight: 27762 (sequence Q25UJ2) |

**Table 2: Other microorganisms described to produce polyphosphate-glucose phosphotransferase enzymatic activity**

| Microorganism | Phosphate donor | Phosphate acceptor | Comments |
|---|---|---|---|
| Arthrobacter sp. | | | Km (glucose): 0.5 mM (pH 7.0; 30°C). |
| | Polyphosphate | D-glucose | Km (hexametaP): 0.02 mM (pH 7.0; 30°C). |
| | | | Specific activity: 220 U/mg. |
| | | | pH opt.: 7.5 |
| | Polyphosphate | D-mannose | About 40% of the activity with glucose. |
| | | | Km (mannose): 0.15 mM (pH 7.0; 30°C). |
| | ATP | D-glucose | Specific activity: 110 U/mg. |
| | ATP | D-mannose | About 30% of the activity with glucose. |
| Corynebacterium diphtheriae | Polyphosphate | D-glucose | 253 amino acids; mol. weight: 27024 (sequence Q6NGU6) |
| Corynebacterium glutamicum | Polyphosphate | D-glucose | 276 amino acids; mol. weight: 29645 (sequence Q8NPA4) |
| Corynebacterium xerosis | Polyphosphate | D-glucose | |
| Microlunatus phosphovorus | | | pH opt.: 5.5 |
| | Polyphosphate | D-glucose | Polyphosphate accumulating bacterium |
| | | | (Tanaka et al. J Bact, 2003 185: 5654 - 5656). Dimer: 2 x 32000 |
| | Polyphosphate | D-mannose | Slow reaction. |
| | Polyphosphate | Glucosamine | Rate similar to that of glucose. |
| | ATP | D-glucose | No activity with ATP. |
| Nocardiaminima | ATP | D-glucose | NaCI above 100 mM inhibits. Mol. weight: 59000 |
| Propionibacterium arabinosum | ATP | D-glucose | Dimer: 2 x 30000 Turnover: 63 s⁻¹ |
| | Polyphosphate | D-glucose | Turnover: 55 s⁻¹ |
| Propionibacterium freudenreichii shermanii | Polyphosphate | D-glucose | 264 amino acids; mol. weight: 27788 (sequence Q3LF16) |
| Propionibacterium shermanii | | | Km glucose: 1.1 mM (pH 7.4). |
| | | | Km (PolyP): 0.94 (PP40), 0.013 (PP75), 5 10⁻⁶ (PP400) (pH 7.5; 30°C). |
| | | | Turnover (PP400): 371 s⁻¹. |
| | Polyphosphate | D-glucose | Turnover (PP35): 57 s⁻¹. |
| | | | Specific activity: 15.3 U/mg. |
| | | | Constitutive enzyme involved in metabolism of glucose. |
| | | | Monomer (31000) |
| | | | ADP and Glc6P inhibit. |
| | | | D-xylose inhibits. |
| | ATP | D-glucose | TriPoly(P) inhibits. |
| | | | Km glucose: 1.1 mM (pH 7.4). |
| | | | Km (ATP): 1.5 mM (pH 7.5; 30°C). |
| | | | Turnover: 25 s⁻¹. |
| | CTP | D-glucose | Km (CTP): 5.9 mM (pH 7.5; 30°C). |
| | dATP | D-glucose | |
| Rubrobacter xylanophilus DSM 9941 | Polyphosphate | D-glucose | 266 amino acids; mol. weight: 28039 (sequence available) |

As shown in these two tables, and on the basis of the reported investigations dealing with the discovery and exploitation of the polyphosphate-glucose phosphotransferase enzymatic activity, the following main points appear:
- several enzymes that recognize polyphosphate as a phosphate donor have been discovered and tested,
- all these enzymes recognize D-glucose as acceptor when polyphosphate is used as a phosphate donor: for both reasons, all of these enzymes have been gathered in a specific group of enzymes (namely, the group EC 2.7.1.63) whereas the vast majority of the phosphotransferases that transfer a phosphate group from a donor to an alcohol use ATP (adenosine triphosphate) as donor (sub-class EC 2.7.1 according to the recommendations of the Nomenclature Committee of the International Union of Biochemistry and Molecular Biology on the Nomenclature and Classification of Enzymes by the reactions they catalyse), and
- among all these enzymes, beside glucose, only mannose or glucosamine are recognized by only three polyphosphate-glucose phosphotransferases; mannose and glucosamine are phosphorylated at the primary alcohol by the enzymes produced by Arthrobacter sp. and Microlunatus phosphovorus and it is reported that the Mycobacterium phlei polyphosphate-glucose phosphotransferase is able to produce glucosamine-6-phosphate beside glucose-6-phosphate (Szymona M. Utilization of inorganic polyphosphates for phosphorylation of glucose in Mycobacterium phlei, Bull Acad Pol Sci Ser Sci Biol, 1957 5: 379 - 381).
   In addition, the Mycobacterium phlei polyphosphate-glucotransferase is up to now the only one that has been investigated with the perspective of a larger scale preparation of glucose-6-phosphate (Girbal E, Binot R, Monsan P. Production, purification, properties and kinetic studies of free and immobilized polyphosphate: glucose-6-phosphotransferase from Mycobacterium phlei, Enzyme Microb Technol, 1989 11: 518 - 527).
   As previously mentioned, the only known substances able to act as phosphate acceptor for this enzyme are glucose and glucosamine: Szymona (Szymona M. Utilization of inorganic polyphosphates for phosphorylation of glucose in Mycobacterium phlei, Bull Acad Pol Sci Ser Sci Biol, 1957 5: 379 - 381) states that the Mycobacterium phlei polyphosphate-glucose phosphotransferase has rigourous substrate specificity with no effect on other hexoses, pentoses and nucleosides. The identification of new phosphate acceptors seems nevertheless to depend on the mode of culture of the bacterial cells: a polyphosphate-fructose phosphotransferase (fructose is an hexose with a ketonic carbonyl or potential ketonyl carbonyl, the ketonic carbonyl being at C-2) was present when the bacterial cells were grown in the presence of fructose (Szymona O, Szumillo T. Adenosine triphosphate and inorganic polyphosphate fructokinases of Mycobacterium phlei, Acta Biochim Pol, 1966 28: 153 - 160). Szymona et al (Szymona O, Kowalska H, Szymona M. Search for inducible sugar kinases in Mycobacterium phlei, Ann Univ Mariae Curie-Sklodowska Sect D, 1969 24: 1 - 18) report the discovery of two other adaptative enzymes: polyphosphate-mannose phosphotransferase was obtained when the bacterial cells were grown on mannose and polyphosphate-gluconic acid phosphotransferase in the presence of a specific substrate. As far as the Arthrobacter sp. strain KM is concerned, the polyphosphate phosphotransferase secreted is able to phosphorylate the primary alcohol group of glucose and mannose (activity ratio of 3:2) but fructose, galactose, glucosamine and 2-deoxyglucose are inert as phosphoryl acceptors irrespective of the phosphoryl donor, polyphosphate or ATP (Mukai T, Kawai S, Matsukawa H, Matuo Y, Murata K. Characterization and molecular cloning of a novel enzyme, inorganic polyphosphate / ATP - glucomannokinase, of Arthrobacter sp. strain KM, Applied Environ Microbiol, 2003 69 (7): 3849 - 3857).

To date, it is therefore clear that, as far as aldohexoses and aldohexoses derivatives are concerned, there is a need to provide a method implementing a biocatalyst able to recognize a large set of phosphate acceptors and a cheap source of phosphate to obtain aldohexoses phosphorylated at the primary alcohol group, except for glucose, mannose and glucosamine.

There is therefore a need to obtain various phosphorylated aldohexose derivatives in a simple and cost effective way and according to an industrial feasible process.

The invention is based on the discovery that polyphosphate-glucose phosphotransferase can have other specific phosphate acceptors than the conventional phosphate acceptors, i.e. D-glucose, D-mannose and glucosamine.

### SUMMARY OF THE INVENTION

The present invention concerns a method for preparing C-6 phosphorylated D-aldohexoses and C-6 phosphorylated D-aldohexose derivatives.

The obtained C-6 phosphorylated D-aldohexoses and C-6 phosphorylated D-aldohexose derivatives can be used directly, in the form of an acid or in the form of a salt (sodium, potassium or any other inorganic or organic cationic substance), or as synthesis intermediates for dermocosmetics preparations, fine chemistry specialties, food and feed application, detergents compositions, and products for therapeutic or diagnostic purposes.

The present invention further deals with novel C-6 phosphorylated D-aldohexoses and derivatives thereof, which can be obtained by the method according to the invention, and uses thereof, including as medicaments.

The invention further relates to disaccharides, polysaccharides, chemical compounds or polymers derived from the enzymatic or chemical polymerization of one or several phosphorylated D-aldohexose derivatives of the present invention, and uses thereof, including as medicaments.

The present invention also concerns a composition, preferably a pharmaceutical or cosmetic composition, comprising a phosphorylated aldohexose derivative of the present invention or a disaccharide, polysaccharide, chemical compound or polymer obtained from enzymatic or chemical polymerization of one or several said phosphorylated aldohexose derivatives of the present invention.

The present invention also concerns the use of a phosphorylated D-aldohexose or D-aldohexose derivative of the present invention or a disaccharide, polysaccharide, chemical compound or polymer obtained from enzymatic or chemical polymerization of one or several said phosphorylated D-aldohexose or D-aldohexose derivative for preparing a pharmaceutical or cosmetic composition for treating a cancer, a cardiovascular disease, a bacterial infection, a UVB-induced erythema, an allergy, an inflammatory or immune disorder, a knee pain, or an osteoarthritis.

The present invention also concerns the use of a phosphorylated aldohexose derivative of the present invention or a disaccharide, polysaccharide, chemical compound or polymer obtained from enzymatic or chemical polymerization of one or several of said phosphorylated aldohexose derivatives as nutritional supplements.

The present invention also concerns the use of a phosphorylated aldohexose derivative of the present invention or a disaccharide, polysaccharide, chemical compounds or polymer obtained from enzymatic or chemical polymerization of one or several of said phosphorylated aldohexose derivatives as detergents or moisturizers.

### DETAILED DESCRIPTION OF THE INVENTION

The method of the present invention comprises reacting polyphosphate, as sole source of phosphate donor, and a polyphosphate-glucose phosphotransferase (polyphosphate: D-glucose 6-phosphotransferase, EC 2.7.1.63) in an aqueous solution with a D-aldohexose or a D-aldohexose derivative having the general formula (1): 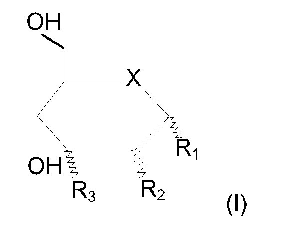 wherein:
R₁ is selected from the group consisting of OH, SH, and H;
X is selected from the group consisting of O, NH, and S;
R₂ and R₃ are positioned as follows:
   both below or above, or R₂ is below and R₃ is above,
R₂ represents H, OH, OCH₃, NH₂, NHCOCH₃, NHSO₃H group or an halogen atom, and
R₃ represents H, OH, OCH₃ group or an halogen atom,
with the following provisos:
when R₁ is OH, X is an oxygen atom, R₂ and R₃ are both above, then R₂ and R₃ are not both OH; and
when R₁ is OH, X is an oxygen atom, R₂ is below and R₃ is above and represents OH, then R₂ is not OH or NH₂ group;
or any salt thereof.

Formula (I) corresponds to a Haworth projection of monosaccharides deriving from D-erythrose. Haworth projection as defined below is a common way of representing the cyclic structure of monosaccharides with a simple three-dimensional perspective (carbon and hydrogen atoms of the carbon chain are not represented therein).

Compounds of formula (I) include compounds wherein R1 is below (α), above (β) the ring or mixture thereof.

### DEFINITIONS

### Carbohydrates, monosaccharides and D-aldohexoses structure

Carbohydrates are organic compounds that typically contain hydrogen and oxygen in a ratio 2:1 and thus have the general empirical formula Cₓ(H₂O)_{y}. The designation "carbohydrates" is also used for oxidation and reduction products as well as simple derivatives of these compounds such as amino carbohydrates (an alcoholic hydroxy group is replaced by an amino group) and the esters of carbohydrates with phosphoric acid (an alcoholic hydroxy group is replaced by a phosphate group).
The generic term "monosaccharide" denotes a carbohydrate that cannot be split further by hydrolysis. Indeed, the other members of the carbohydrate family of substances are oligosaccharides and polysaccharides which are composed of monosaccharides or their derivatives linked together by glycosidic linkages. These glycosidic linkages (see later for the definition of the glycosidic linkage) can be split by hydrolysis, e.g. in acid media or with specific enzymes. The term "oligosaccharide" is commonly used to refer to a defined structure as opposed to a polymer, named "polysaccharide", of unspecified length and containing a large number of monosaccharide residues (Mac Naught AD. Nomenclature of carbohydrates, Pure & Appl Chem, 1996 68: 1919 - 2008).

Chemically, monosaccharides are polyhydroxy aldehydes or polyhydroxy ketones, respectively H-[CHOH]ₙ-CHO and H-[CHOH]ₙ-CO-[CHOH]ₘ, with 3 to 9 carbon atoms: the former, which contain an aldehyde carbonyl or potential aldehydic carbonyl group (the term "potential aldehydic carbonyl group" refers to the hemiacetal group arising from ring closure - see below for details), are named "aldoses" for "aldehyde" and the conventional ending "-ose" for carbohydrates, and the later, which contain a ketonic carbonyl or potential ketonic carbonyl group (the term "potential ketonic carbonyl group" refers to the hemiketal group arising from ring closure), are named "ketoses" for "ketone" and the conventional ending "-ose" for carbohydrates. Monosaccharides are simply depicted (Fischer representation) in the form of an open chain written vertically according to convention, with the carbonyl group at the upper end. The carbon atoms are numbered from top to bottom and the groups projecting left and right of the carbon chain are considered as being in front of the plane of the paper.
Aldohexoses are monosaccharides having six (this justifies the infix "-hex-") carbon atoms and an aldehydic carbonyl group. From a structural point of view, they are considered to be derived from the simplest stable carbohydrate, glyceraldehyde: CH₂OH-CHOH-CHO. Its carbon atom in position 2 according to the Fischer representation (C-1 is that of CHO and C-2 is that of CHOH) is asymmetric (chiral) and an isomerism accompanied by optical activity results from this asymmetry: one isomer rotates the plane of polarized light to the right (it is said "dextro" or "d" - in italicized letters - or (+)) and the other one rotates the plane of polarized light to the left (it is said "levo" or "I" or (-)). The classification of aldohexose molecules is based on their relationship to glyceraldehyde: with the carbonyl group at the upper end (Fischer representation), the hydroxyl linked to the carbon atom in position 2 is located at the right of the carbon chain in dextrorotatory glyceraldehyde and the hydroxyl linked to the carbon atom in position 2 is located at the left of the carbon chain in levorotatory glyceraldehyde. Aldohexoses are considered to be derived from dextro- or levo-rotatory glyceraldehyde by 3 repeated insertions of secondary alcohol groups (CHOH) at the carbonyl group: the carbon atom of the carbonyl remains the C-1 carbon atom, the adjacent carbon atom is the C-2 carbon atom and the carbon atom in the CH₂OH group is the C-6 carbon atom (carbon atom in the CH₂OH group with the number 6). With each additional asymmetric center, the number of possible isomers increases and the direction of rotation of polarized light of the resulting substance described by the symbols (+) and (-) in no way indicates whether the latter is derived from dextro- or levorotatory glyceraldehyde. The relationship between the substance and glyceraldehyde is given by the symbols D and L (in small-capital roman letters): all those monosaccharides and related compounds in which the secondary alcohol group farthest removed from the principal functional group, aldehyde in the case of aldoses, has the same steric configuration as the dextrorotatory glyceraldehyde are assigned to the D series (all those monosaccharides and related compounds in which the secondary alcohol group farthest removed from the aldehyde group has the same steric configuration as the levorotatory glyceraldehyde are assigned to the L series). The monosaccharides occurring naturally belong, with few exceptions, to the D series.

The aldohexose compounds can exist in straight-chain and cyclic forms, the latter, which is predominant at neutral pH, being the result of an intramolecular reaction between the carbonyl group and the C-5 hydroxyl group to form an intramolecular hemiacetal. Indeed, aldoses with more than four carbon atoms in the chain do not behave as normal aldehydes in that they will not color Schiff's reagent nor produce acetal reaction with methanolic hydrogen chloride. In addition, the optical rotation of their freshly prepared aqueous solutions, which is the result of the presence of asymmetric carbon atoms, changes rapidly until it reaches a new constant value; this process is called mutarotation and reflects the presence of two isomers unexpected from the linear or open chain structure of monosaccharides as proposed by Fischer. The new centre of chirality generated by the hemiacetal ring closure is called the anomeric centre. The two stereoisomers of a cyclic monosaccharide molecule arising during ring closure are referred as anomers and denoted by the symbols α and β, the designation α being given to the isomer in the D-series that is more strongly dextrorotatory, and β to the isomer in the L-series that is more strongly levorotatory. Deriving from that of Fischer, the Fischer-Tollens projection represents the cyclic form of monosaccharides with the oxygen bridge formed during cyclization at the right-hand side of the carbon chain (conventional writing). The hydroxyl group resulting from cyclization is written to the right of the newly formed asymmetric carbon atom when the isomer rotates more strongly to the right (α-D or β-L configuration) and to the left thereof when the isomer rotates more strongly to the left (β-D or α-L configuration). The Haworth projection derives from that of Fischer-Tollens as follows: the monosaccharide is depicted with the carbon-chain horizontal and in the plane of the paper, the carbonyl group involved in ring closure being to the right. At the asymmetric carbon involved via oxygen in ring formation with the carbonyl group, a formal double inversion is carried out to have the attached hydroxyl group in the prolongation of the carbon chain (when considering D-glucose represented by a six-membered ring, the CH₂OH replaces the H atom and the H atom takes the place of the CH₂OH group in the first inversion, and the H atom and the OH group exchange their position in the second inversion). The oxygen bridge is then depicted as being formed behind the plane of the paper. The heterocyclic ring is formed, taking into consideration that the newly formed centre of chirality is in the plane of the paper and that the oxygen atom is behind the plane of the paper. The Haworth representation is thus a perspective drawing of a simplified model: the ring is oriented almost perpendicular to the plane of the paper, but viewed from slightly above so that the edge closest to the viewer is drawn below the more distant edge, with the oxygen behind. To help to define the perspective, the ring bonds closer to the viewer are often thickened but this is not a rule. The carbon atom from the carbonyl group is at the right-hand end and the ring atoms are numbered clockwise and designed C-1 (the carbon atom of the carbonyl group), C-2 and so on. Groups that appear to the right of the Fischer or the Fischer-Tollens projections appear below the plane of the ring, those on the left appear above. In the common representation of the six-membered ring form of D-aldohexoses, C-6 is above the plane. For simplicity, the carbon atoms and the hydrogen atoms that are located at each corner of the hexagon are not shown. As far as the anomeric centre is concerned, the hydroxyl group resulting from cyclization is written below the plane of the ring in the case of the α-D anomer and above the plane in the case of the β-D anomer. The α and β isomers yield the corresponding α- and β-glycosides: this is of importance because many hydrolytic enzymes are α- or β-specific. As suggested earlier when depicting monosaccharides as carbohydrate that can not be split further by hydrolysis, monosaccharides can be linked to other monosaccharides through a glycosidic link: a glycosidic link is formed when the hemiacetal (or hemiketal) hydroxy group of a carbohydrate reacts with the hydroxy group of an other carbohydrate (or an other compound) with elimination of water; the resulting compound is called a glycoside.
The monosaccharide representation was further improved in order to take into account the non planar structure of their cyclized form: the description of the arrangement in space of the various atoms is not necessary in the present context to describe unambiguously the monosaccharides and monosaccharide derivatives related to Formula (I).
As noted above, the preferred structural form of aldohexoses is that of a cyclic hemiacetal. Five and six-membered rings are favored over other ring sizes from a thermodynamic point of view, and among them, the preferred form is a six-membered ring.

**Table 3: position of various groups of the common aldohexose D-glucose in the Fischer, Fischer-Tollens (pyranose form) and Haworth (pyranose form) projections**

| Projection | C-1 | OH on C-2 | OH on C-3 | OH on C-4 | C-5 | CH₂OH |
|---|---|---|---|---|---|---|
| Fischer | Top of carbon chain (vertical) | Right-hand side (RHS) of carbon chain | Left-hand side (LHS) of carbon chain | RHS of carbon chain | OH: right-hand side of carbon chain | Bottom of carbon chain |
| Fischer-Tollens | Newly formed OH by cyclization: α : RHS β :LHS | RHS of carbon chain | LHS of carbon chain | RHS of carbon chain | Oxygen bridge: right-hand side of carbon chain | Left-hand side of carbon chain |
| Haworth | Newly formed OH by cyclization: α : below the ring β ; above the ring | Below the ring | Above the ring | Below the ring | The ring bond between C-5 and oxygen corresponds to the back of the ring | Above the ring |

As stated earlier, aldoses with at least four carbon atoms are considered to be derived from glyceraldehyde by repeated insertion of secondary alcohol groups from dextrorotatory ((+) or d) glyceraldehyde for those of the D-series and from levorotatory ((-) or I) glyceraldehyde for those of the L-series. As a consequence, D-aldohexoses can be considered to be derived from (+)-glyceraldehyde by repeated insertion of 3 secondary alcohol groups: the number possible of isomers obtained after the 3 repeated insertions of secondary alcohol groups is equal to 8 (2³; in total, there are 4 centres of asymmetry and thus 16 optical isomers can be listed (2⁴): 8 of the D-series and 8 of the L-series). Table 4 reports the list of the 8 aldohexoses with the position of the various groups of the D-monosaccharides according to the Haworth (pyranose form) projection. The name reported in the first column is the trivial name of each substance. The corresponding systematic names are as follows:
- the symbol D or L (small capital letter) indicating the relationship with (+) or (-) glyceraldehyde respectively is followed by a hyphen;
- the said hyphen is followed by the configurational prefix in italicized letters: the configurational prefix is the trivial name minus the two last letters "se" (gluco for glucose etc...); the said configurational prefix is followed by a hyphen;
- the said hyphen is followed by the stem name which is related to the number of carbon atoms: hexose is the stem name of the monosaccharides containing 6 carbon atoms.
   The systematic name can be enriched by the symbols α and β when the nature of the isomer is known and by the suffix pyranose or furanose when the size of the ring is known (an exemple is α-D-gluco-hexopyranose).

**Table 4: list of the 8 aldohexoses with the position of the various groups of the D-monosaccharides according to the Haworth (pyranose form) projection**

| D-Aldohexose | C-1 | OH on C-2 | OH on C-3 | OH on C-4 | C-5 | CH₂OH |
|---|---|---|---|---|---|---|
| D-Allose | Newly | Below* | Below* | Below* | The ring | |
| D-Altrose | formed OH | Above* | Below* | Below* | bond | |
| D-Glucose | by | Below* | Above* | Below* | between C- | |
| D-Mannose | cyclization: | Above* | Above* | Below* | 5 and | Above the |
| D-Gulose | α: below | Below* | Below* | Above* | oxygen | ring |
| D-ldose | the ring | Above* | Below* | Above* | corresponds | |
| D-Galactose | β: above | Below* | Above* | Above* | to the back of | |
| D-Talose | the ring | Above* | Above* | Above* | the ring | |

| | | | | | | |
|---|---|---|---|---|---|---|
| *: Below or Above the ring | | | | | | |

According to the invention, D-Galactose, D-Gulose, D-ldose, D-Talose, D-Mannose, D-Glucose and D-Altrose of table 4 are excluded from formula (I).

As far as monosaccharide derivatives are concerned, their naming has to be performed in accordance with the recommendations of IUPAC and IUBMB (Mac Naught AD. ibid).

### Enzyme

Enzyme is any protein molecule that catalyses chemical reactions on molecules (named substrates) to obtain other molecules (named products). A recommended name, i.e. a systematic name which stresses the type of reaction that the enzyme is able to catalyze and an Enzyme Commission (EC) code number (according to the recommendations of the nomenclature committee of the International Union of Biochemistry on the Nomenclature and Classification of Enzyme-Catalysed Reactions), is assigned to each enzyme. These code numbers, prefixed by EC, contain four elements separated by points. The first number shows to which of the six main divisions (classes) the enzyme belongs: oxidoreductases (EC 1), transferases (EC 2), hydrolases (EC 3), lyases (EC 4), isomerases (EC 5) and ligases (EC 6). The second number indicates the subclass, the third the sub-subclass and the fourth is the serial number of the enzyme in its sub-subclass.

### Polyphosphate

Polyphosphate is a salt or ester of polyphosphoric acid having the general formula [PO₃H]ₙ, wherein n is higher than 3 (i.e., polymerization degree higher than 3), and preferably less than 30, and more preferably less than 20. Polyphosphate is usually available in the form of a salt of sodium. The polyphosphate has preferably a polymerization degree of at least 4, preferably ranging from 8 to 20 and conventionaly named Graham salts.

Polyphosphate-glucose phosphotransferases (EC 2.7.1.63): an enzyme that can use polyphosphate to phosphorylate D-glucose at the C-6 position to create D-glucose-6-phosphate.

Where "comprising" is used, this can preferably be replaced by "consisting essentially of", more preferably by "consisting of".

Within the context of the invention, the term "treatment" denotes curative, symptomatic, and preventive treatment. Compounds of the invention can be used in humans with existing disease, including at early or late stages of progression of the disease. The compounds of the invention will not necessarily cure the patient who has the disease but will delay or slow the progression or prevent further progression of the disease, ameliorating thereby the patients' condition. The compounds of the invention can also be administered to those who do not have the diseases but who would normally develop the disease or be at increased risk for the disease, they will not develop the disease. Treatment also includes delaying the development of the disease in an individual who will ultimately develop the disease or would be at risk for the disease due to age, familial history, genetic or chromosomal abnormalities, and/or due to the presence of one or more biological markers for the disease, such as a known genetic mutation in tissues or fluids. By delaying the onset of the disease, compounds of the invention have prevented the individual from getting the disease during the period in which the individual would normally have gotten the disease or reduce the rate of development of the disease or some of its effects but for the administration of compounds of the invention up to the time the individual ultimately gets the disease. Treatment also includes administration of the compounds of the invention to those individuals thought to be predisposed to the disease. In treating the above diseases, the compounds of the invention are administered in a therapeutically effective amount.

### PREFERRED EMBODIMENTS

### Aldohexoses or derivatives thereof and their phosphorylation

Aldohexoses or derivatives thereof which can be used in the method according to the invention are represented by formula (I). Taking into consideration the conventional Haworth representation of monosaccharides, their parent aldotetraose is D-erythrose (the conventional insertion of HCOH to D-glyceraldehyde gives D-erythrose whereas the insertion of HOCH gives D-threose; the conventional insertion of HCOH to D-erythrose gives D-ribose whereas the insertion of HOCH to D-erythrose gives D-arabinose). The D-aldohexoses of Formula (I) can be obtained by conventional insertion of secondary hydroxyl groups to D-ribose and D-arabinose.

A non exhaustive list of aldohexose derivatives is reported in Table 5.

**Table 5: list of derivatives that can be phosphorylated at the primary alcohol group.**

| | | | |
|---|---|---|---|
| Parent | D-glucose | D-mannose | D-allose |
| monosaccharide | R2=OH: below the ring | R2=OH: above the ring | R2=OH: below the ring |
| | R3=OH: above the ring | R3=OH: above the ring | R3=OH: below the ring |
| X = O and R1 = OH and R3 = OH | | | |
| R2: H | 2-deoxy-D-glucose | 2-deoxy-D-mannose | 2-deoxy-D-allose |
| R2: OCH₃ | 2-O-methyl-D-glucose | 2-O-methyl-D-mannose | 2-O-methyl-D-allose |
| R2: F | 2-fluoro-2-deoxy-D-glucose | 2-fluoro-2-deoxy-D-mannose | 2-fluoro-2-deoxy-D-allose |
| R2: NH₂ | D-glucosamine (2-amino-2-deoxy-D-glucose) | D-mannosamine (2-amino-2-deoxy-D-mannose) | D-allosamine (2-amino-2-deoxy-D-allose) |
| R2: NHCOCH₃ | N-acetyl-D-glucosamine | N-acetyl-D-mannosamine | N-acetyl-D-allosamine |
| X = O and R1 = OH and R2 = OH | | | |
| R3: OCH₃ | 3-O-methyl-D-glucose | 3-O-methyl-D-mannose | 3-O-methyl-D-allose |
| X = O and R1 = H | | | |
| R2 and R3: OH | 1,5 anhydro-D-glucitol | 1,5 anhydro-D-mannitol | 1,5 anhydro-D-allitol |
| X = O and R1 = SH | | | |
| R2 and R3: OH | 1-thio-D-glucose | 1-thio-D-mannose | 1-thio-D-allose |
| X = S and R1 = OH | | | |
| R2 and R3: OH | 5-thio-D-glucose | 5-thio-D-mannose | 5-thio-D-allose |
| X = NH and R1 = OH | | | |
| R2 and R3: OH | 5-amino-D-glucose (or nojirimycin) | 5-amino-D-mannose (or mannojirimycin) | 5-amino-D-allose (or allonojirimycin) |

As defined in formula (I), D-glucose, D-mannose and D-glucosamine are excluded by the provisos.

According to the invention, compounds of formula (I) include any inorganic or organic salt thereof as identified below (for the obtained phosphorylated compounds), in particular inorganic salt such as sodium or potassium.
The halogen atom includes F, Cl, Br or I, and more particularly F or Cl.
In a specific embodiment, the D-aldohexose or a D-aldohexose derivative has the general formula (I) wherein R₂ and R₃ are both below.
In another specific embodiment, the D-aldohexose or a D-aldohexose derivative has the general formula (I) wherein R₂ and R₃ are both above.
In another specific embodiment, the D-aldohexose or a D-aldohexose derivative has the general formula (I) wherein R₂ is below and R₃ is above. According to this specific embodiment, a more specific embodiment is where R₃ is H, OCH₃, or an halogen atom, and more specifically where R₂ is OH.
In yet another specific embodiment, the D-aldohexose or a D-aldohexose derivative has the general formula (I) wherein X is O.

The D-aldohexoses or derivatives thereof which are more particularly used in the present invention are selected in the group consisting of the compounds identified in the following tables 6a-6f and any salt thereof.

**Table 6a: X = O and R1 = OH**

| R2 | R3 | Name |
|---|---|---|
| OH | OH | D-allose |
| | H | 3-deoxy-D-(glucose or mannose or allose) |
| | OCH₃ | 3-O-methyl-D-(glucose or mannose or allose) |
| | F or Cl or Br or | 3-deoxy-3-(fluoro or chloro or bromo or iodo)-D-(glucose or mannose or allose) |
| H | OH | 2-deoxy-D-(mannose or allose) |
| | H | 2,3-dideoxy-D-(mannose or allose) |
| | OCH₃ | 3-O-methyl-2-deoxy-D-(glucose or mannose or allose) |
| | F or Cl or Br or I | 3-deoxy-3-(fluoro or chloro or bromo or iodo)-2-deoxy-D-(glucose or mannose or allose) |
| OCH₃ | OH | 2-O-methyl-D-(glucose or mannose or allose) |
| | H | 3-deoxy-2-O-methyl-D-(glucose or mannose or allose) |
| | OCH₃ | 2,3-di-O-methyl-D-(glucose or mannose or allose) |
| | F or Cl or Br or | 3-deoxy-3-(fluoro or chloro or bromo or iodo)-2-O-methyl-D-(glucose or mannose or allose) |
| NH₂ | OH | D-(mannosamine or allosamine) |
| | H | 2,3-dideoxy-2-amino-D-(glucose or mannose or allose) |
| | OCH₃ | 3-O-methyl-D-(glucosamine or mannosamine or allosamine) |
| | F or Cl or Br or | 3-deoxy-3-(fluoro or chloro or bromo or iodo)-D-(glucosamine or mannosamine or allosamine) |
| NHCOCH₃ | OH | N-acetyl-D-(glucosamine or mannosamine or allosamine) |
| | H | 2,3-dideoxy-2-acetamido-D-(glucose or mannose or allose) |
| | OCH₃ | 3-O-methyl-N-acetyl-D-(glucosamine or mannosamine or allosamine) |
| | F or Cl or Br or | 3-deoxy-3-(fluoro or chloro or bromo or iodo)-N-acetyl-D-(glucosamine or mannosamine or allosamine) |
| NHSO₃H | OH | 2-deoxy-2-sulfamino-D-(glucose or mannose or allose) |
| | H | 2,3-dideoxy-2-sulfamino-D-(glucose or mannose or allose) |
| | OCH₃ | 3-O-methyl-2-deoxy-2-sulfamino-D-(glucose or mannose or allose) |
| | F or Cl or Br or | 2,3-dideoxy-3-(fluoro or chloro or bromo or iodo)-2-sulfamino-D-(glucose or mannose or allose) |
| F or Cl or Br or I | OH | 2-deoxy-2-(fluoro or chloro or bromo or iodo)-D-(glucose or mannose or allose) |
| | H | 2,3-dideoxy-2-(fluoro or chloro or bromo or iodo)-D-(glucose or mannose or allose) |
| | OCH₃ | 3-O-methyl-2-deoxy-2-(fluoro or chloro or bromo or iodo)-D-(glucose or mannose or allose) |
| | F or Cl or Br or | 2,3-dideoxy-2,3-(fluoro or chloro or bromo or iodo)-D-(glucose or mannose or allose) |

**Table 6b: X = O and R1 = H**

| R2 | R3 | Name |
|---|---|---|
| OH | OH | 1,5-anhydro-D-(glucitol or mannitol or allitol) |
| | H | 3-deoxy-1,5-anhydro-D-(glucitol or mannitol or allitol) |
| | OCH₃ | 3-O-methyl-1,5-anhydro-D-(glucitol or mannitol or allitol) |
| | F or Cl or Br or | 3-deoxy-3-(fluoro or chloro or bromo or iodo)-1,5-anhydro-D-(glucitol or mannitol or allitol) |
| H | OH | 2-deoxy-1,5-anhydro-D-(glucitol or mannitol or allitol) |
| | H | 2,3-dideoxy-1,5-anhydro-D-(glucitol or mannitol or allitol) |
| | OCH₃ | 3-O-methyl-2-deoxy-1,5-anhydro-D-(glucitol or mannitol or allitol) |
| | F or Cl or Br or | 2,3-deoxy-3-(fluoro or chloro or bromo or iodo)-1,5-anhydro-D-(glucitol or mannitol or allitol) |
| OCH₃ | OH | 2-O-methyl-1,5-anhydro-D-(glucitol or mannitol or allitol) |
| | H | 3-deoxy-2-O-methyl-1 ,5-anhydro-D-(glucitol or mannitol or allitol) |
| | OCH₃ | 2,3-di-O-methyl-1,5-anhydro-D-(glucitol or mannitol or allitol) |
| | F or Cl or Br or I | 3-deoxy-3-(fluoro or chloro or bromo or iodo)-2-O-methyl-1,5-anhydro-D-(glucitol or mannitol or allitol) |
| NH₂ | OH | 2-deoxy-2-amino-1,5-anhydro-D-(glucitol or mannitol or allitol) |
| | H | 2,3-dideoxy-2-amino-1,5-anhydro-D-(glucitol or mannitol or allitol) |
| | OCH₃ | 3-O-methyl-2-deoxy-2-amino-1,5-anhydro-D-(glucitol or mannitol or allitol) |
| | F or Cl or Br or I | 2,3-dideoxy-3-(fluoro or chloro or bromo or iodo)-2-amino-1,5-anhydro-D-(glucitol or mannitol or allitol) |
| NHCOCH₃ | OH | 2-deoxy-2-acetamido-1,5-anhydro-D-(glucitol or mannitol or allitol)-6-phosphate |
| | H | 2,3-dideoxy-2-acetamido-1,5-anhydro-D-(glucitol or mannitol or allitol) |
| | OCH₃ | 3-O-methyl-2-deoxy-2-acetamido-1,5-anhydro-D-(glucitol or mannitol or allitol) |
| | F or Cl or Br or I | 2,3-dideoxy-3-(fluoro or chloro or bromo or iodo)-2-acetamido-1,5-anhydro-D-(glucitol or mannitol or allitol) |
| NHSO₃H | OH | 2-deoxy-2-sulfamino-1,5-anhydro-D-(glucitol or mannitol or allitol) |
| | H | 2,3-dideoxy-2-sulfamino-1,5-anhydro-D-(glucitol or mannitol or allitol) |
| | OCH₃ | 3-O-methyl-2-deoxy-2-sulfamino-1,5-anhydro-D-(glucitol or mannitol or allitol) |
| | F or Cl or Br or I | 2,3-dideoxy-3-(fluoro or chloro or bromo or iodo)-2-sulfamino-1,5-anhydro-D-(glucitol or mannitol or allitol) |
| F or Cl or Br or I | OH | 2-deoxy-2-(fluoro or chloro or bromo or iodo)-1,5-anhydro-D-(glucitol or mannitol or allitol) |
| | H | 2,3-dideoxy-2-(fluoro or chloro or bromo or iodo)-1,5-anhydro-D-(glucitol or mannitol or allitol) |
| | OCH₃ | 3-O-methyl-2-deoxy-2-(fluoro or chloro or bromo or iodo)-1,5-anhydro-D-(glucitol or mannitol or allitol) |
| | F or Cl or Br or I | 2,3-dideoxy-2,3-(fluoro or chloro or bromo or iodo)-1,5-anhydro-D-(glucitol or mannitol or allitol) |

**Table 6c: X = O and R1 = SH**

| R2 | R3 | Name |
|---|---|---|
| OH | OH | D-glucothiose or D-mannothiose or D-allothiose |
| | H | 3-deoxy-D-(glucothiose or mannothiose or allothiose) |
| | OCH₃ | 3-O-methyl-D-(glucothiose or mannothiose or allothiose) |
| | F or Cl or Br or I | 3-deoxy-3-(fluoro or chloro or bromo or iodo)-D-(glucothiose or mannothiose or allothiose) |
| H | OH | 2-deoxy-D-(glucothiose or mannothiose or allothiose) |
| | H | 2,3-dideoxy-D-(glucothiose or mannothiose or allothiose) |
| | OCH₃ | 3-O-methyl-2-deoxy-D-(glucothiose or mannothiose or allothiose) |
| | F or Cl or Br or | 3-deoxy-3-(fluoro or chloro or bromo or iodo)-2-deoxy-D-(glucothiose or mannothiose or allothiose) |
| OCH₃ | OH | 2-O-methyl-D-(glucothiose or mannothiose or allothiose) |
| | H | 3-deoxy-2-O-methyl-D-(glucothiose or mannothiose or allothiose) |
| | OCH₃ | 2,3-di-O-methyl-D-(glucothiose or mannothiose or allothiose) |
| | F or Cl or Br or | 3-deoxy-3-(fluoro or chloro or bromo or iodo)-2-O-methyl-D-(glucothiose or mannothiose or allothiose) |
| NH₂ | OH | 1-thio-D-(glucosamine or mannosamine or allosamine) |
| | H | 2,3-dideoxy-2-amino-D-(glucothiose or mannothiose or allothiose) |
| | OCH₃ | 3-O-methyl-1-thio-D-(glucosamine or mannosamine or allosamine) |
| | F or Cl or Br or | 3-deoxy-3-(fluoro or chloro or bromo or iodo)-1-thio-D-(glucosamine or mannosamine or allosamine) |
| NHCOCH₃ | OH | N-acetyl-1-thio-D-(glucosamine or mannosamine or allosamine) |
| | H | 2,3-dideoxy-2-acetamido-D-(glucothiose or mannothiose or allothiose) |
| | OCH₃ | 3-O-methyl-N-acetyl-1-thio-D-(glucosamine or mannosamine or allosamine) |
| | F or Cl or Br or | 3-deoxy-3-(fluoro or chloro or bromo or iodo)-N-acetyl-1-thio-D-(glucosamine or mannosamine or allosamine) |
| NHSO₃H | OH | 2-deoxy-2-sulfamino-D-(glucothiose or mannothiose or allothiose) |
| | H | 2,3-dideoxy-2-sulfamino-D-(glucothiose or mannothiose or allothiose) |
| | OCH₃ | 3-O-methyl-2-deoxy-2-sulfamino-D-(glucothiose or mannothiose or allothiose) |
| | F or Cl or Br or | 2,3-dideoxy-3-(fluoro or chloro or bromo or iodo)-2-sulfamino-D-(glucothiose or mannothiose or allothiose) |
| F or Cl or Br or I | OH | 2-deoxy-2-(fluoro or chloro or bromo or iodo)-D-(glucothiose or mannothiose or allothiose) |
| | H | 2,3-dideoxy-2-(fluoro or chloro or bromo or iodO)-D-(glucothiose or mannothiose or allothiose) |
| | OCH₃ | 3-O-methyl-2-deoxy-2-(fluoro or chloro or bromo or iodo)-D-(glucothiose or mannothiose or allothiose) |
| | F or Cl or Br or | 2,3-dideoxy-2,3-(fluoro or chloro or bromo or iodo)-D-(glucothiose or mannothiose or allothiose) |

**Table 6d: X = S and R1 = OH**

| R2 | R3 | Name |
|---|---|---|
| OH | OH | 5-thio-D-glucose or 5-thio-D-mannose or 5-thio-D-allose |
| | H | 3-deoxy-5-thio-D-(glucose or mannose or allose) |
| | OCH₃ | 3-O-methyl-5-thio-D-(glucose or mannose or allose) |
| | F or Cl or Br or I | 3-deoxy-3-(fluoro or chloro or bromo or iodo)-5-thio-D-(glucose or mannose or allose) |
| H | OH | 2-deoxy-5-thio-D-(glucose or mannose or allose) |
| | H | 2,3-dideoxy-5-thio-D-(glucose or mannose or allose) |
| | OCH₃ | 3-O-methyl-2-deoxy-5-thio-D-(glucose or mannose or allose) |
| | F or Cl or Br or | 2,3-dideoxy-3-(fluoro or chloro or bromo or iodo)-5-thio-D-(glucose or mannose or allose) |
| OCH₃ | OH | 2-O-methyl-5-thio-D-(glucose or mannose or allose) |
| | H | 3-deoxy-2-O-methyl-5-thio-D-(glucose or mannose or allose) |
| | OCH₃ | 2,3-di-O-methyl-5-thio-D-(glucose or mannose or allose) |
| | F or Cl or Br or I | 3-deoxy-3-(fluoro or chloro or bromo or iodo)-2-O-methyl-5-thio-D-(glucose or mannose or allose) |
| NH₂ | OH | 5-thio-D-(glucosamine or mannosamine or allosamine) |
| | H | 2,3-dideoxy-2-amino-5-thio-D-(glucose or mannose or allose) |
| | OCH₃ | 3-O-methyl-5-thio-D-(glucosamine or mannosamine or allosamine) |
| | F or Cl or Br or | 3-deoxy-3-(fluoro or chloro or bromo or iodo)-5-thio-D-(glucosamine or mannosamine or allosamine) |
| NHCOCH₃ | OH | N-acetyl-5-thio-D-(glucosamine or mannosamine or allosamine) |
| | H | 2,3-dideoxy-2-acetamido-5-thio-D-(glucose or mannose or allose) |
| | OCH₃ | 3-O-methyl-N-acetyl-5-thio-D-(glucosamine or mannosamine or allosamine) |
| | F or Cl or Br or | 3-deoxy-3-(fluoro or chloro or bromo or iodo)-N-acetyl-5-thio-D-(glucosamine or mannosamine or allosamine) |
| NHSO₃H | OH | 2-deoxy-2-sulfamino-5-thio-D-(glucose or mannose or allose) |
| | H | 2,3-dideoxy-2-sulfamino-5-thio-D-(glucose or mannose or allose) |
| | OCH₃ | 3-O-methyl-2-deoxy-2-sulfamino-5-thio-D-(glucose or mannose or allose) |
| | F or Cl or Br or | 2,3-dideoxy-3-(fluoro or chloro or bromo or iodo)-2-sulfamino-5-thio-D-(glucose or mannose or allose) |
| F or Cl or Br or I | OH | 2-deoxy-2-(fluoro or chloro or bromo or iodo)-5-thio-D-(glucose or mannose or allose) |
| | H | 2,3-dideoxy-2-(fluoro or chloro or bromo or iodo)-5-thio-D-(glucose or mannose or allose) |
| | OCH₃ | 3-O-methyl-2-deoxy-2-(fluoro or chloro or bromo or iodo)-5-thio-D-(glucose or mannose or allose) |
| | F or Cl or Br or | 2,3-dideoxy-2,3-(fluoro or chloro or bromo or iodo)-5-thiO-D-(glucose or mannose or allose) |

**Table 6e: X = NH and R1 = OH**

| R2 | R3 | Name |
|---|---|---|
| OH | OH | 5-deoxy-5-amino-D-glucose or 5-amino-D-mannose or 5-amino-D-allose |
| | H | 3,5-dideoxy-5-amino-D-(glucose or mannose or allose) |
| | OCH₃ | 3-O-methyl-5-deoxy-5-amino-D-(glucose or mannose or allose) |
| | F or Cl or Br or | 3,5-dideoxy-3-(fluoro or chloro or bromo or iodo)-5-amino-D-(glucose or mannose or allose) |
| H | OH | 2,5-dideoxy-5-amino-D-(glucose or mannose or allose) |
| | H | 2,3,5-trideoxy-5-amino-D-(glucose or mannose or allose) |
| | OCH₃ | 3-O-methyl-2,5-dideoxy-5-amino-D-(glucose or mannose or allose) |
| | F or Cl or Br or | 2,3,5-trideoxy-3-(fluoro or chloro or bromo or iodo)-5-amino-D-glucose or mannose or allose) |
| OCH₃ | OH | 2-O-methyl-5-deoxy-5-amino-D-(glucose or mannose or allose) |
| | H | 3,5-dideoxy-2-O-methyl-5-amino-D-(glucose or mannose or allose) |
| | OCH₃ | 2,3-di-O-methyl-5-deoxy-5-amino-D-(glucose or mannose or allose) |
| | F or Cl or Br or I | 3,5-dideoxy-3-(fluoro or chloro or bromo or iodo)-2-O-methyl-5-amino-D-(glucose or mannose or allose) |
| NH₂ | OH | 5-deoxy-5-amino-D-(glucosamine or mannosamine or allosamine) |
| | H | 2,3,5-trideoxy-2,5-diamino-D-(glucose or mannose or allose) |
| | OCH₃ | 3-O-methyl-5-deoxy-5-amino-D-(glucosamine or mannosamine or allosamine) |
| | F or Cl or Br or I | 3,5-dideoxy-3-(fluoro or chloro or bromo or iodo)-5-amino-D-(glucosamine or mannosamine or allosamine) |
| NHCOCH₃ | OH | 2,5-dideoxy-5-amino-2-acetamido-D-(glucose or mannose or allose) |
| | H | 2,3,5-trideoxy-2-acetamido-5-amino-D-(glucose or mannose or allose) |
| | OCH₃ | 3-O-methyl-2-acetamido-2,5-dideoxy-5-amino-D-(glucose or mannose or allose) |
| | F or Cl or Br or I | 2,3,5-trideoxy-3-(fluoro or chloro or bromo or iodo)-2-acetamido-5-amino-D-(glucose or mannose or allose) |
| NHSO₃H | OH | 2,5-dideoxy-2-sulfamino-5-amino-D-(glucose or mannose or allose) |
| | H | 2,3,5-trideoxy-2-sulfamino-5-amino-D-(glucose or mannose or allose) |
| | OCH₃ | 3-O-methyl-2,5-dideoxy-2-sulfamino-5-amino-D-(glucose or mannose or allose) |
| | F or Cl or Br or I | 2,3,5-trideoxy-3-(fluoro or chloro or bromo or iodo)-2-sulfamino-5-amino-D-(glucose or mannose or allose) |
| F or Cl or Br or I | OH | 2,5-dideoxy-2-(fluoro or chloro or bromo or iodo)-5-amino-D-(glucose or mannose or allose) |
| | H | 2,3,5-trideoxy-2-(fluoro or chloro or bromo or iodo)-5-amino-D-(glucose or mannose or allose) |
| | OCH₃ | 3-O-methyl-2,5-dideoxy-2-(fluoro or chloro or bromo or iodo)-5-amino-D-(glucose or mannose or allose) |
| | F or Cl or Br or I | 2,3,5-trideoxy-2,3-(fluoro or chloro or bromo or iodo)-5-amino-D-(glucose or mannose or allose) |

**Table 6f: X = NH and R1 = H**

| R2 | R3 | Name |
|---|---|---|
| OH | OH | 1,5-dideoxy-5-amino-D-(glucose or mannose or allose) |
| | H | 1,3,5-trideoxy-5-amino-D-(glucose or mannose or allose) |
| | OCH₃ | 3-O-methyl-1,5-dideoxy-5-amino-D-(glucose or mannose or allose) |
| | F or Cl or Br or I | 1,3,5-trideoxy-3-(fluoro or chloro or bromo or iodo)-5-aminO-D-(glucose or mannose or allose) |
| H | OH | 1,2,5-trideoxy-5-amino-D-(glucose or mannose or allose) |
| | H | 1,2,3,5-tetradeoxy-5-amino-D-(glucose or mannose or allose) |
| | OCH₃ | 3-O-methyl-1,2,5-trideoxy-5-amino-D-(glucose or mannose or allose) |
| | F or Cl or Br or I | 1,2,3,5-tetradeoxy-3-(fluoro or chloro or bromo or iodo)-5-amino-D-glucose or mannose or allose) |
| OCH₃ | OH | 2-O-methyl-1,5-dideoxy-5-amino-D-(glucose or mannose or allose |
| | H | 1,3,5-trideoxy-2-O-methyl-5-amino-D-(glucose or mannose or allose) |
| | OCH₃ | 2,3-di-O-methyl-1,5-dideoxy-5-amino-D-(glucose or mannose or allose) |
| | F or Cl or Br or I | 1,3,5-trideoxy-3-(fluoro or chloro or bromo or iodo)-2-O-methyl-5-amino-D-(glucose or mannose or allose) |
| NH₂ | OH | 1,5-dideoxy-5-amino-D-(glucosamine or mannosamine or allosamine) |
| | H | 1,2,3,5-tetradeoxy-2,5-diamino-D-(glucose or mannose or allose) |
| | OCH₃ | 3-O-methyl-1,5-dideoxy-5-amino-D-(glucosamine or mannosamine or allosamine) |
| | F or Cl or Br or I | 1,3,5-trideoxy-3-(fluoro or chloro or bromo or iodo)-5-amino-D-(glucosamine or mannosamine or allosamine) |
| NHCOCH₃ | OH | 1,2,5-trideoxy-5-amino-2-acetamido-D-(glucose or mannose or allose) |
| | H | 1,2,3,5-tetradeoxy-2-acetamido-5-amino-D-(glucose or mannose or allose) |
| | OCH₃ | 3-O-methyl-2-acetamido-1,2,5-trideoxy-5-amino-D-(glucose or mannose or allose) |
| | F or CI or Br or I | 1,2,3,5-tetradeoxy-3-(fluoro or chloro or bromo or iodo)-2-acetamido-5-amino-D-(glucose or mannose or allose) |
| NHSO₃H | OH | 1,2,5-trideoxy-2-sulfamino-5-amino-D-(glucose or mannose or allose) |
| | H | 1,2,3,5-tetraeoxy-2-sulfamino-5-amino-D-(glucose or mannose or allose) |
| | OCH₃ | 3-O-methyl-1,2,5-trideoxy-2-sulfamino-5-amino-D-(glucose or mannose or allose) |
| | F or Cl or Br or I | 1,2,3,5-tetradeoxy-3-(fluoro or chloro or bromo or iodo)-2-sulfamino-5-amino-D-(glucose or mannose or allose) |
| F or Cl or Br or I | OH | 1,2,5-trideoxy-2-(fluoro or chloro or bromo or iodo)-5-amino-D-(glucose or mannose or allose) |
| | H | 1,2,3,5-tetradeoxy-2-(fluoro or chloro or bromo or iodo)-5-amino-D-(glucose or mannose or allose) |
| | OCH₃ | 3-O-methyl-1,2,5-trideoxy-2-(fluoro or chloro or bromo or iodo)-5-amino-D-(glucose or mannose or allose) |
| | F or Cl or Br or I | 1,2,3,5-tetradeoxy-2,3-(fluoro or chloro or bromo or iodo)-5-amino-D-(glucose or mannose or allose) |

The D-aldohexose or derivative thereof of formula (I) are more particularly selected in the group consisting of D-allose, 2-deoxy-D-glucose, 2-deoxy-D-mannose, 2-deoxy-D-allose, 2-O-methyl-D-glucose, 2-O-methyl-D-mannose, 2-O-methyl-D-allose, 2-fluoro-2-deoxy-D-glucose, 2-fluoro-2-deoxy-D-mannose, 2-fluoro-2-deoxy-D-allose, D-mannosamine, D-allosamine, N-acetyl-D-glucosamine, N-acetyl-D-mannosamine, N-acetyl-D-allosamine, 3-O-methyl-D-glucose, 3-O-methyl-D-mannose, 3-O-methyl-D-allose, 1,5 anhydro-D-glucitol, 1,5 anhydro-D-mannitol, 1,5 anhydro-D-allitol, 1-thio-D-glucose, 1-thio-D-mannose, 1-thio-D-allose, 5-thio-D-glucose, 5-thio-D-mannose, 5-thio-D-allose, 5-amino-D-glucose, 5-amino-D-mannose, 5-amino-D-allose, 2-deoxy-2-sulfamino-D-glucose, 2-deoxy-2-sulfamino-D-mannose, 2-deoxy-2-sulfamino-D-allose and salts thereof.

The above identified compounds exist either in nature or have been obtained by any convenient techniques (most of them can be found in fine chemicals listings such as those of SIGMA-ALDRICH-FLUKA, and ACROS ORGANICS).

### Source of the enzyme

The enzymes that are used in this phosphorylation reaction are phosphotransferases with an alcohol group as acceptor, more specifically the enzymes are polyphosphate-glucose phosphotransferases (EC 2.7.1.63) as defined above.
In a preferred embodiment, the enzyme used for the desired phosphorylation of D-aldohexose or derivative thereof with polyphosphate is a polyphosphate-glucose phosphotransferase from a bacterial species, more particularly from a Mycobacterium species, and more preferably from Mycobacterium tuberculosis (H₃₇Rv), Mycobacterium smegmatis (mc²155), Mycobacterium phlei, Mycobacterium bovis, Mycobacterium avium paratuberculosis, Mycobacterium flavescens, Mycobacterium leprae, Mycobacterium sp. JLS, Mycobacterium sp. KMS, or Mycobacterium vanbaalenii, Mycobacterium aurum, Mycobacterium gordonae, Mycobacterium jucho, Mycobacterium pellegrino, or Mycobacterium thermorestibile. In a most preferred embodiment, polyphosphate-glucose phosphotransferase is from Mycobacterium tuberculosis (H₃₇Rv), Mycobacterium smegmatis (mc²155), Mycobacterium phlei, Mycobacterium bovis, Mycobacterium avium paratuberculosis, Mycobacterium flavescens, Mycobacterium leprae, Mycobacterium sp. JLS, Mycobacterium sp. KMS, or Mycobacterium vanbaalenii.

Alternative sources of enzyme may be polyphosphate-glucose phosphotransferases derived from Arthrobacter sp., Corynebacterium sp., Microlunatus sp., Mycobacterium sp., Nocardia sp., Propionibacterium sp., Streptomyces sp., Rhodococcus sp., or Bifidobacterium sp..

Such enzymes can be obtained by a natural fermentation of the producing strains, followed by cell treatments and enzyme recovery and purification. Since polyphosphate-glucose phosphotransferases are mainly intracellular large enzymes, the techniques that can be used for the recovery of the enzyme include but are not limited to cells recovery, techniques aiming at cell disruption include but are not limited to French press homogeneization, glass beads, sonification or any equivalent method. The techniques aiming at enzyme concentration include but are not limited to ultrafiltration with a molecular weight cut off ranging from 5 kDa to 300 kDa, and the techniques that can be used for enzyme purification include but are not limited to phase partition with polyethylene glycol, gel permeation chromatography, molecular separation with ultrafiltration membranes of cut off ranging from 10 to 30 kDa.
As several sequences of either validated or putative polyphosphate-glucose phosphotransferases are contained in public databases, an alternative solution consists in the recombinant expression of said enzymes in well known expression hosts such as E. coli, S. cerevisiae, Baculovirus, Y. lipolytica, Bacillus sp., Pseudomonas sp., H. polymorpha or mammalian cells (see as one reference "Production of Recombinant Proteins: Novel Microbial and Eukaryotic Expression Systems" Ville Wiley 2004 - Gerd Gellissen Ed.), optionally followed by (a) purification step(s) using well known methods by one in the art. More precisely, the polyphosphate-glucose phosphotransferases can be expressed as described for the polyphosphate glucotransferase of M. tuberculosis (Hsieh PC, Shenoy BC, Samols D, Philips NFB. Cloning, expression and characterization of polyphosphate glucokinase from Mycobacterium tuberculosis, J Biol Chem, 1996 271 (9): 4909 - 4915). Such recombinant expression approach does not only allow to rely on a more efficient and cost effective production of the enzyme, but it also enables to avoid the manipulation of native strains when they are pathogenic to humans, animals or plants.
A non exhaustive list of the currently available sequences of polyphosphate-glucose phosphotransferases correspond to genes of the following bacteriae:
- Mycobacterium bovis (AF2122/97 gi 31619300_155994)
- Mycobacterium avium paratuberculosis (AAS05136)
- Mycobacterium flavescens PYR-GCK (gi_89317556-c94830-94027)
- Mycobacterium leprae (TN NC002677)
- Mycobacterium smegmatis (MC2 TIGR MSMEG2760)
- Mycobacterium sp. JLS (ctg24 gi_92434752_704148-704957)
- Mycobacterium sp. KMS (ctg57 (MCS) gi_92439887_c131460-130651)
- Mycobacterium tuberculosis H₃₇Rv (NC000962)
- Mycobacterium vanbaalenii PYR-1 (ctg22 gi 90205262_c256405-255587)
- putative Bifidobacterium breve (gi_57233099_1-765)
- Rhodococcus fascians (gi_18477826_89-913)
- Streptomyces avermitilis (MA-4680 gi_57833846_4000786-4001532)
- Streptomyces coelicolor A3(2) (gi_24413879_129055-129795).

One should notice that these genes show conserved domains corresponding to phosphate binding domain or glucose binding domains. Therefore degenerated primers can be designed to rapidly discover and clone other polyphosphate-glucose phosphotransferases genes.

The enzyme can also be obtained through well known methods from one in the art: random mutagenesis, directed mutagenesis or directed evolution methods (MIYAZAKI K, ARNOLD FH, (2004), In vitro DNA recombination. In Phage Display: A practical approach. Clarkson T and Lowman H, editors. New York:Orxford University Press Inc., 43-60). These technologies can enable to obtain enzymes with higher specific activity, lower products inhibition, dedicated region, chemio and stereo selectivity, better stability, or any combination thereof.

The method of the invention can thus be carried out with either whole cells, eventually after a treatment aiming at facilitating the accessibility of the substrates to the enzyme and/or facilitating the excretion of the products, or with a natural or recombinant crude or purified enzyme.
The enzyme can be used under its "free" form, or as an immobilized catalyst. Such immobilisation procedures include but are not limited to gel encapsulation (calcium alginate, ...) more preferably when the biocatalyst is a whole cell, resin adsorption, glutaraldehyde reticulation, spray drying in the presence eventually of an adequate adjuvant to obtain an insoluble form of the enzyme, ultrafiltration membrane reactors (filtration when the biocatalyst is a whole cell) or any combination thereof, and are well known in the art. The choice of one immobilisation approach relies on its economical cost and on the final yield of the process involving said immobilized enzyme.
The enzymatic activity of an enzyme preparation can be assessed by using a reference assay in which glucose is used as a substrate, and the amount of synthesized glucose-6-phosphate is revealed with a glucose-6-phosphate dehydrogenase whose cofactor consumption is measured by spectrophotometric UV absorbance analysis. The polyphosphate-glucose phosphotransferase activity is expressed in µmoles (10⁻⁶ moles) of phosphorylated D-aldohexose produced per minute.

### Polyphosphate

The polyphosphate used in the present invention presents a polymerization degree higher than 3, and preferably less than 30, and more preferably less than 20. Polyphosphate are usually available in the form of a salt of sodium. The polyphosphate has preferably a polymerization degree of at least 4, preferably ranging from 8 to 20 and conventionally named Graham salts.

### Reaction conditions

The reaction medium convenient for the synthesis of C-6 phosphorylated D-aldohexoses and D-aldohexose derivatives according to the invention contains polyphosphate-glucose phosphotransferase, the phosphate donor polyphosphate, and the acceptor monosaccharide as defined by formula (I). Preferably, the reaction medium further comprises a magnesium salt, and more particularly magnesium chloride. Optionally, it can also comprise a buffer system in order to ensure a defined pH value to maintain the activity of the polyphosphate-glucose phosphotransferase and to prevent the denaturation of the enzyme during the reaction, optionally any additive that could favour the enzymatic activity and/or stability (as an example, a protease inhibitor if the polyphosphate-glucose phosphotransferase enzyme is contained in a rather poorly purified preparation), and/or optionally any other compound that could induce the separation of the desired product from the reaction medium in order to facilitate its purification and/or the synthesis yield.
The pH of the reaction medium is ajusted in order to favor the enzyme stability and/or activity and the working pH value is the best compromise found in order to achieve the highest synthesis yield and/or highest product concentration in a convenient period of time (from several minutes to a few dozen of hours). The working pH value more preferably ranges from 5.5 to 9.0 and more specifically from 6.0 to 8.5.
The reaction medium comprises an aqueous solution. Preferably, said aqueous solution comprises water and any other convenient additive compound as mentioned above.

The temperature of the reaction medium is preferably maintained in order to favor the enzyme stability and/or activity and the working temperature value is preferably the best compromise found in order to achieve the highest synthesis yield and/or highest product concentration in a convenient period of time (from several minutes to a few dozen of hours). The working temperature value more preferably ranges from 15 to 45°C, and more specifically from 18 to 32°C.

Typically, the carbohydrate or carbohydrate derivative (compound of formula (I)) concentration ranges from 0.05 to 2.0 M, preferably from 0.10 to 1.0 M. Generally, the transferable phosphate concentration varies between 0,05 mole per mole of carbohydrate to 2 moles per mole of carbohydrate (taking into account that the limit polyphosphate oligomer has a polymerization degree of 3, the polyphosphate concentration is then calculated as follows: carbohydrate concentration in M multiplied by the stoichiometry factor multiplied by the average molecular weight of the polyphosphate substrate divided by the average transferable phosphate polymerization degree). The enzymatic activity in the reaction medium is preferably at least 0.05 U/ml and generally does not exceed 5 U/ml.

### Purification

C-6 phosphorylated D-aldohexoses and D-aldohexoses derivatives obtained by the method of the present invention can be used directly or purified to reach a desired purity in terms of residual non C-6 phosphorylated D-aldohexose or D-aldohexose derivatives and/or residual polyphosphate and enzyme.
This purification can be achieved using conventional techniques, including ultrafiltration using mineral or organic membranes for the removal of the biocatalyst when it is in a soluble form. Residual polyphosphate can be removed in the form of insoluble barium or calcium salt after the addition in the reaction mixture of barium or calcium hydroxyde or barium or calcium chloride or barium or calcium carbonate or any other inorganic or organic salt of barium or calcium; anion exchange chromatography can also be used to fix the phosphorylated substances and the substances of interest can be collected using a solution containing at least a negatively charged substance able to interact with the positively charged support and thus to replace the released substance; selective precipitation of the C-6 phosphorylated D-aldohexose or D-aldohexose derivative using a combination of a barium or calcium salt and a water miscible organic solvent such as methanol, ethanol, propanol, isopropanol, acetone, glycol, propylene glycol, glycol ethers,... Purification can be achieved by using other conventional techniques, such as HPLC.

### Novel C-6 phosphorylated D-aldohexoses

The C-6 phosphorylated D-aldohexoses and derivatives obtained by the method according to the invention present the following general formula (II): 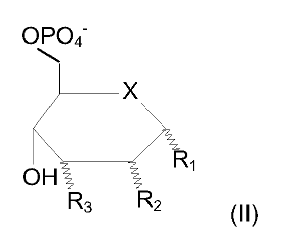 wherein:
R1, R2, R3 and X are as defined in formula (I) or any salt thereof.

According to a specific embodiment, the present invention further deals with novel C-6 phosphorylated D-aldohexoses and derivatives thereof, which can be obtained by the method according to the invention, and uses thereof, including as medicaments.

The C-6 phosphorylated D-aldohexoses and derivatives thereof also include any inorganic and organic salt of the phosphorylated compounds, in particular due to the phosphate group which is an ionisable group.

In a particular embodiment, the invention relates to C-6 phosphorylated D-aldohexoses and derivatives thereof selected in the group consisting of the compounds reported by the following tables 7a-7f and any salt thereof.

Tables 7a-7f report new phosphorylated aldohexoses and phosphorylated aldohexose derivatives compounds obtainable by the method of the invention (non ionized form)

**Table 7a: X = O and R1 = OH**

| R2 | R3 | Name |
|---|---|---|
| OH | H | 3-deoxy-D-(glucose or mannose or allose)-6-phosphate |
| | OCH₃ | 3-O-methyl-D-(allose)-6-phosphate |
| | F or Cl or Br or | 3-deoxy-3-chloro-D-(glucose or mannose or allose)-6-phosphate 3-deoxy-3-fluoro-o-(mannose or allose)-6-phosphate 3-deoxy-3-bromo-D-(glucose or mannose or allose)-6-phosphate 3-deoxy-3-iodO-D-(glucose or mannose or allose)-6-phosphate |
| H | OH | 2-deoxy-D-(mannose or allose)-6-phosphate |
| | H | 2,3-dideoxy-D-(glucose or mannose or allose)-6-phosphate |
| | OCH₃ | 3-O-methyl-2-deoxy-D-(glucose or mannose or allose)-6-phosphate |
| | F or CI or Br or I | 3-deoxy-3-(fluoro or chloro or bromo or iodo)-2-deoxy-D-(glucose or mannose or allose)-6-phosphate |
| OCH₃ | OH | 2-O-methyl-D-allose-6-phosphate |
| | H | 3-deoxy-2-O-methyl-D-(glucose or mannose or allose)-6-phosphate |
| | OCH₃ | 2,3-di-O-methyl-D-(glucose or mannose or allose)-6-phosphate |
| | F or Cl or Br or | 3-deoxy-3-(fluoro or chloro or bromo or iodo)-2-O-methyl-D-(glucose or mannose or allose)-6-phosphate |
| NH₂ | OH | D-allosamine-6-phosphate |
| | H | 2,3-dideoxy-2-amino-D-(glucose or mannose or allose)-6-phosphate |
| | OCH₃ | 3-O-methyl-D-(glucosamine or mannosamine or allosamine)-6-phosphate |
| | F or Cl or Br or | 3-deoxy-3-(fluoro or chloro or bromo or iodo)-D-(glucosamine or mannosamine or allosamine)-6-phosphate |
| NHCOCH₃ | OH | N-acetyl-D-allosamine-6-phosphate |
| | H | 2,3-dideoxy-2-acetamido-D-(glucose or mannose or allose)-6-phosphate |
| | OCH₃ | 3-O-methyl-N-acetyl-D-(glucosamine or mannosamine or allosamine)-6-phosphate |
| | F or Cl or Br or | 3-deoxy-3-(fluoro or chloro or bromo or iodo)-N-acetyl-D-(glucosamine or mannosamine or allosamine)-6-phosphate |
| NHSO₃H | OH | 2-deoxy-2-sulfamino-D-(glucose or mannose or allose)-6-phosphate |
| | H | 2,3-dideoxy-2-sulfamino-D-(glucose or mannose or allose)-6-phosphate |
| | OCH₃ | 3-O-methyl-2-deoxy-2-sulfamino-D-(glucose or mannose or allose)-6-phosphate |
| | F or Cl or Br or | 2,3-dideoxy-3-(fluoro or chloro or bromo or iodo)-2-sulfamino-D-(glucose or mannose or allose)-6-phosphate |
| F or Cl or Br or I | OH | 2-deoxy-2-(fluoro or chloro or bromo or iodo)-D-allose-6-phosphate |
| | H | 2,3-dideoxy-2-(fluoro or chloro or bromo or iodo)-D-(glucose or mannose or allose)-6-phosphate |
| | OCH₃ | 3-O-methyl-2-deoxy-2-(fluoro or chloro or bromo or iodo)-D-(glucose or mannose or allose)-6-phosphate |
| | F or Cl or Br or | 2,3-dideoxy-2,3-(fluoro or chloro or bromo or iodo)-D-(glucose or mannose or allose)-6-phosphate |

**Table 7b: X = O and R1 = H**

| R2 | R3 | Name |
|---|---|---|
| OH | OH | 1,5-anhydro-D-(mannitol or allitol)-6-phosphate |
| | H | 3-deoxy-1,5-anhydro-D-(glucitol or mannitol or allitol)-6-phosphate |
| | OCH₃ | 3-O-methyl-1,5-anhydro-D-(glucitol or mannitol or allitol)-6-phosphate |
| | F or Cl or Br or | 3-deoxy-3-(fluoro or chloro or bromo or iodo)-1,5-anhydro-D-(glucitol or mannitol or allitol)-6-phosphate |
| H | OH | 2-deoxy-1,5-anhydro-D-(glucitol or mannitol or allitol)-6-phosphate |
| | H | 2,3-dideoxy-1,5-anhydro-D-(glucitol or mannitol or allitol)-6-phosphate |
| | OCH₃ | 3-O-methyl-2-deoxy-1,5-anhydro-D-(glucitol or mannitol or allitol)-6-phosphate |
| | F or CI or Br or | 2,3-deoxy-3-(fluoro or chloro or bromo or iodo)-1,5-anhydro-D-(glucitol or mannitol or allitol)-6-phosphate |
| OCH₃ | OH | 2-O-methyl-1,5-anhydro-D-(glucitol or mannitol or allitol)-6-phosphate |
| | H | 3-deoxy-2-O-methyl-1,5-anhydro-D-(glucitol or mannitol or allitol)-6-phosphate |
| | OCH₃ | 2,3-di-O-methyl-1,5-anhydro-D-(glucitol or mannitol or allitol)-6-phosphate |
| | F or Cl or Br or I | 3-deoxy-3-(fluoro or chloro or bromo or iodo)-2-O-methyl-1,5-anhydro-D-(glucitol or mannitol or allitol)-6-phosphate |
| NH₂ | OH | 2-deoxy-2-amino-1,5-anhydro-D-(glucitol or mannitol or allitol)-6-phosphate |
| | H | 2,3-dideoxy-2-amino-1,5-anhydro-D-(glucitol or mannitol or allitol)-6-phosphate |
| | OCH₃ | 3-O-methyl-2-deoxy-2-amino-1,5-anhydro-D-(glucitol or mannitol or allitol)-6-phosphate |
| | F or Cl or Br or I | 2,3-dideoxy-3-(fluoro or chloro or bromo or iodo)-2-amino-1,5-anhydro-D-(glucitol or mannitol or allitol)-6-phosphate |
| NHCOCH₃ | OH | 2-deoxy-2-acetamido-1,5-anhydro-D-(glucitol or mannitol or allitol)-6-phosphate |
| | H | 2,3-dideoxy-2-acetamido-1,5-anhydro-D-(glucitol or mannitol or allitol)-6-phosphate |
| | OCH₃ | 3-O-methyl-2-deoxy-2-acetamido-1 ,5-anhydro-D-(glucitol or mannitol or allitol)-6-phosphate |
| | F or Cl or Br or I | 2,3-dideoxy-3-(fluoro or chloro or bromo or iodo)-2-acetamido-1,5-anhydro-D-(glucitol or mannitol or allitol)-6-phosphate |
| NHSO₃H | OH | 2-deoxy-2-sulfamino-1,5-anhydro-D-(glucitol or mannitol or allitol)-6-phosphate |
| | H | 2,3-dideoxy-2-sulfamino-1,5-anhydro-D-(glucitol or mannitol or allitol)-6-phosphate |
| | OCH₃ | 3-O-methyl-2-deoxy-2-sulfamino-1,5-anhydro-D-(glucitol or mannitol or allitol)-6-phosphate |
| | F or Cl or Br or I | 2,3-dideoxy-3-(fluoro or chloro or bromo or iodo)-2-sulfamino-1,5-anhydro-D-(glucitol or mannitol or allitol)-6-phosphate |
| F or Cl or Br or I | OH | 2-deoxy-2-(fluoro or chloro or bromo or iodo)-1,5-anhydro-D-(glucitol or mannitol or allitol)-6-phosphate |
| | H | 2,3-dideoxy-2-(fluoro or chloro or bromo or iodo)-1,5-anhydro-D-(glucitol or mannitol or allitol)-6-phosphate |
| | OCH₃ | 3-O-methyl-2-deoxy-2-(fluoro or chloro or bromo or iodo)-1,5-anhydro-D-(glucitol or mannitol or allitol)-6-phosphate |
| | F or Cl or Br orI | 2,3-dideoxy-2,3-(fluoro or chloro or bromo or iodo)-1,5-anhydro-D-(glucitol or mannitol or allitol)-6-phosphate |

**Table 7c: X = O and R1 = SH**

| R2 | R3 | Name |
|---|---|---|
| OH | OH | D-glucothiose-6-phosphate or D-mannothiose-6-phosphate or D-allothiose-6-phosphate |
| | H | 3-deoxy-D-(glucothiose or mannothiose or allothiose)-6-phosphate |
| | OCH₃ | 3-O-methyl-D-(glucothiose or mannothiose or allothiose)-6-phosphate |
| | F or Cl or Br or | 3-deoxy-3-(fluoro or chloro or bromo or iodo)-D-(glucothiose or mannothiose or allothiose)-6-phosphate |
| H | OH | 2-deoxy-D-(glucothiose or mannothiose or allothiose)-6-phosphate |
| | H | 2,3-dideoxy-D-(glucothiose or mannothiose or allothiose)-6-phosphate |
| | OCH₃ | 3-O-methyl-2-deoxy-D-(glucothiose or mannothiose or allothiose)-6-phosphate |
| | F or Cl or Br or | 3-deoxy-3-(fluoro or chloro or bromo or iodo)-2-deoxy-D-I (glucothiose or mannothiose or allothiose)-6-phosphate |
| OCH₃ | OH | 2-O-methyl-D-(glucothiose or mannothiose or allothiose)-6-phosphate |
| | H | 3-deoxy-2-O-methyl-D-(glucothiose or mannothiose or allothiose)-6-phosphate |
| | OCH₃ | 2,3-di-O-methyl-D-(glucothiose or mannothiose or allothiose)-6-phosphate |
| | F or Cl or Br or | 3-deoxy-3-(fluoro or chloro or bromo or iodo)-2-O-methyl-D-(glucothiose or mannothiose or allothiose)-6-phosphate |
| NH₂ | OH | 1-thio-D-(glucosamine or mannosamine or allosamine)-6-phosphate |
| | H | 2,3-dideoxy-2-amino-D-(glucothiose or mannothiose or allothiose)-6-phosphate |
| | OCH₃ | 3-O-methyl-1-thio-D-(glucosamine or mannosamine or allosamine)-6-phosphate |
| | F or Cl or Br or | 3-deoxy-3-(fluoro or chloro or bromo or iodo)-1-thio-D-(glucosamine or mannosamine or allosamine)-6-phosphate |
| NHCOCH₃ | OH | N-acetyl-1-thio-D-(glucosamine or mannosamine or allosamine)-6-phosphate |
| | H | 2,3-dideoxy-2-acetamido-D-(glucothiose or mannothiose or allothiose)-6-phosphate |
| | OCH₃ | 3-O-methyl-N-acetyl-1-thio-D-(glucosamine or mannosamine or allosamine)-6-phosphate |
| | F or Cl or Br or | 3-deoxy-3-(fluoro or chloro or bromo or iodo)-N-acetyl-1-thio-D-(glucosamine or mannosamine or allosamine)-6-phosphate |
| NHSO₃H | OH | 2-deoxy-2-sulfamino-D-(glucothiose or mannothiose or allothiose)-6-phosphate |
| | H | 2,3-dideoxy-2-sulfamino-D-(glucothiose or mannothiose or allothiose)-6-phosphate |
| | OCH₃ | 3-O-methyl-2-deoxy-2-sulfaminO-D-(glucothiose or mannothiose or allothiose)-6-phosphate |
| | F or Cl or Br or | 2,3-dideoxy-3-(fluoro or chloro or bromo or iodo)-2-sulfamino-D-(glucothiose or mannothiose or allothiose)-6-phosphate |
| F or Cl or Br or I | OH | 2-deoxy-2-(fluoro or chloro or bromo or iodo)-D-(glucothiose or mannothiose or allothiose)-6-phosphate |
| | H | 2,3-dideoxy-2-(fluoro or chloro or bromo or iodo)-D-(glucothiose or mannothiose or allothiose)-6-phosphate |
| | OCH₃ | 3-O-methyl-2-deoxy-2-(fluoro or chloro or bromo or iodo)-D-(glucothiose or mannothiose or allothiose)-6-phosphate |
| | F or Cl or Br or | 2,3-dideoxy-2,3-(fluoro or chloro or bromo or iodo)-D-(glucothiose or mannothiose or allothiose)-6-phosphate |

**Table 7d: X = S and R1 = OH**

| R2 | R3 | Name |
|---|---|---|
| OH | OH | 5-thio-D-mannose-6-phosphate or 5-thio-D-allose-6-phosphate |
| | H | 3-deoxy-5-thio-D-(glucose or mannose or allose)-6-phosphate |
| | OCH₃ | 3-O-methyl-5-thio-D-(glucose or mannose or allose)-6-phosphate |
| | F or Cl or Br or I | 3-deoxy-3-(fluoro or chloro or bromo or iodo)-5-thio-D-(glucose or mannose or allose)-6-phosphate |
| H | OH | 2-deoxy-5-thio-D-(glucose or mannose or allose)-6-phosphate |
| | H | 2,3-dideoxy-5-thio-D-(glucose or mannose or allose)-6-phosphate |
| | OCH₃ | 3-O-methyl-2-deoxy-5-thio-D-(glucose or mannose or allose)-6-phosphate |
| | F or Cl or Br or | 2,3-dideoxy-3-(fluoro or chloro or bromo or iodo)-5-thio-D-(glucose or mannose or allose)-6-phosphate |
| OCH₃ | OH | 2-O-methyl-5-thio-D-(glucose or mannose or allose)-6-phosphate |
| | H | 3-deoxy-2-O-methyl-5-thio-D-(glucose or mannose or allose)-6-phosphate |
| | OCH₃ | 2,3-di-O-methyl-5-thio-D-(glucose or mannose or allose)-6-phosphate |
| | F or Cl or Br or I | 3-deoxy-3-(fluoro or chloro or bromo or iodo)-2-O-methyl-5-thio-D-(glucose or mannose or allose)-6-phosphate |
| NH₂ | OH | 5-thio-D-(glucosamine or mannosamine or allosamine)-6-phosphate |
| | H | 2,3-dideoxy-2-amino-5-thio-D-(glucose or mannose or allose)-6-phosphate |
| | OCH₃ | 3-O-methyl-5-thio-D-(glucosamine or mannosamine or allosamine)-6-phosphate |
| | F or Cl or Br or | 3-deoxy-3-(fluoro or chloro or bromo or iodo)-5-thio-D-(glucosamine or mannosamine or allosamine)-6-phosphate |
| NHCOCH₃ | OH | N-acetyl-5-thio-D-(glucosamine or mannosamine or allosamine)-6-phosphate |
| | H | 2,3-dideoxy-2-acetamido-5-thio-D-(glucose or mannose or allose)-6-phosphate |
| | OCH₃ | 3-O-methyl-N-acetyl-5-thio-D-(glucosamine or mannosamine or allosamine)-6-phosphate |
| | F or Cl or Br or | 3-deoxy-3-(fluoro or chloro or bromo or iodo)-N-acetyl-5-thio-D-(glucosamine or mannosamine or allosamine)-6-phosphate |
| NHSO₃H | OH | 2-deoxy-2-sulfamino-5-thio-D-(glucose or mannose or allose)-6-phosphate |
| | H | 2,3-dideoxy-2-sulfamino-5-thio-D-(glucose or mannose or allose)-6-phosphate |
| | OCH₃ | 3-O-methyl-2-deoxy-2-sulfamino-5-thio-D-(glucose or mannose or allose)-6-phosphate |
| | F or Cl or Br or I | 2,3-dideoxy-3-(fluoro or chloro or bromo or iodo)-2-sulfamino-5-thio-D-(glucose or mannose or allose)-6-phosphate |
| F or CI or Br or I | OH | 2-deoxy-2-(fluoro or chloro or bromo or iodo)-5-thio-D-(glucose or mannose or allose)-6-phosphate |
| | H | 2,3-dideoxy-2-(fluoro or chloro or bromo or iodo)-5-thio-D-(glucose or mannose or allose)-6-phosphate |
| | OCH₃ | 3-O-methyl-2-deoxy-2-(fluoro or chloro or bromo or iodo)-5-thio-D-(glucose or mannose or allose)-6-phosphate |
| | F or CI or Br or | 2,3-dideoxy-2,3-(fluoro or chloro or bromo or iodo)-5-thio-D-(glucose or mannose or allose)-6-phosphate |

**Table 7e: X = NH and R1 = OH**

| R2 | R3 | Name |
|---|---|---|
| OH | OH | 5-amino-D-allose-6-phosphate |
| | H | 3,5-dideoxy-5-amino-D-(glucose or mannose or allose)-6-phosphate |
| | OCH₃ | 3-O-methyl-5-deoxy-5-amino-D-(glucose or mannose or allose)-6-phosphate |
| | F or Cl or Br or | 3,5-dideoxy-3-(fluoro or chloro or bromo or iodo)-5-amino-D-(glucose or mannose or allose)-6-phosphate |
| H | OH | 2,5-dideoxy-5-amino-D-(glucose or mannose or allose)-6-phosphate |
| | H | 2,3,5-trideoxy-5-amino-D-(glucose or mannose or allose)-6-phosphate |
| | OCH₃ | 3-O-methyl-2,5-dideoxy-5-amino-D-(glucose or mannose or allose)-6-phosphate |
| | F or CI or Br or | 2,3,5-trideoxy-3-(fluoro or chloro or bromo or iodo)-5-amino-D-glucose or mannose or allose)-6-phosphate |
| OCH₃ | OH | 2-O-methyl-5-deoxy-5-amino-D-(glucose or mannose or allose)-6-phosphate |
| | H | 3,5-dideoxy-2-O-methyl-5-amino-D-(glucose or mannose or allose)-6-phosphate |
| | OCH₃ | 2,3-di-O-methyl-5-deoxy-5-amino-D-(glucose or mannose or allose)-6-phosphate |
| | F or CI or Br or I | 3,5-dideoxy-3-(fluoro or chloro or bromo or iodo)-2-O-methyl-5-amino-D-(glucose or mannose or allose)-6-phosphate |
| NH₂ | OH | 5-deoxy-5-amino-D-(glucosamine or mannosamine or allosamine)-6-phosphate |
| | H | 2,3,5-trideoxy-2,5-diamino-D-(glucose or mannose or allose)-6-phosphate |
| | OCH₃ | 3-O-methyl-5-deoxy-5-amino-D-(glucosamine or mannosamine or allosamine)-6-phosphate |
| | F or CI or Br or | 3,5-dideoxy-3-(fluoro or chloro or bromo or iodo)-5-aminO-D-(glucosamine or mannosamine or allosamine)-6-phosphate |
| NHCOCH₃ | OH | 2,5-dideoxy-5-amino-2-acetamido-D-(glucose or mannose or allose)-6-phosphate |
| | H | 2,3,5-trideoxy-2-acetamido-5-amino-D-(glucose or mannose or allose)-6-phosphate |
| | OCH₃ | 3-O-methyl-2-acetamido-2,5-dideoxy-5-amino-D-(glucose or mannose or allose)-6-phosphate |
| | F or CI or Br or I | 2,3,5-trideoxy-3-(fluoro or chloro or bromo or iodo)-2-acetamido-5-amino-D-(glucose or mannose or allose)-6-phosphate |
| NHSO₃H | OH | 2,5-dideoxy-2-sulfamino-5-amino-D-(glucose or mannose or allose)-6-phosphate |
| | H | 2,3,5-trideoxy-2-sulfamino-5-amino-D-(glucose or mannose or allose)-6-phosphate |
| | OCH₃ | 3-O-methyl-2,5-dideoxy-2-sulfamino-5-amino-D-(glucose or mannose or allose)-6-phosphate |
| | F or Cl or Br or I | 2,3,5-trideoxy-3-(fluoro or chloro or bromo or iodo)-2-sulfamino-5-amino-D-(glucose or mannose or allose)-6-phosphate |
| F or Cl or Br or I | OH | 2,5-dideoxy-2-(fluoro or chloro or bromo or iodo)-5-amino-D-(glucose or mannose or allose)-6-phosphate |
| | H | 2,3,5-trideoxy-2-(fluoro or chloro or bromo or iodo)-5-amino-D-(glucose or mannose or allose)-6-phosphate |
| | OCH₃ | 3-O-methyl-2,5-dideoxy-2-(fluoro or chloro or bromo or iodo)-5-amino-D-(glucose or mannose or allose)-6-phosphate |
| | F or Cl or Br or | 2,3,5-trideoxy-2,3-(fluoro or chloro or bromo or iodo)-5-amino-D-(glucose or mannose or allose)-6-phosphate |

**Table 7f: X = NH and R1 = H**

| R2 | R3 | Name |
|---|---|---|
| OH | OH | 1,5-dideoxy-5-amino-D-(glucose or mannose or allose)-6-phosphate |
| | H | 1,3,5-trideoxy-5-amino-D-(glucose or mannose or allose)-6-phosphate |
| | OCH₃ | 3-O-methyl-1,5-dideoxy-5-amino-D-(glucose or mannose or allose)-6-phosphate |
| | F or CI or Br or | 1,3,5-trideoxy-3-(fluoro or chloro or bromo or iodo)-5-amino-D-(glucose or mannose or allose)-6-phosphate |
| H | OH | 1,2,5-trideoxy-5-amino-D-(glucose or mannose or allose)-6-phosphate |
| | H | 1,2,3,5-tetradeoxy-5-amino-D-(glucose or mannose or allose)-6-phosphate |
| | OCH₃ | 3-O-methyl-1,2,5-trideoxy-5-amino-D-(glucose or mannose or allose)-6-phosphate |
| | F or Cl or Br or | 1,2,3,5-tetradeoxy-3-(fluoro or chloro or bromo or iodo)-5-amino-D-glucose or mannose or allose)-6-phosphate |
| OCH₃ | OH | 2-O-methyl-1,5-dideoxy-5-amino-D-(glucose or mannose or allose)-6-phosphate |
| | H | 1,3,5-trideoxy-2-O-methyl-5-amino-D-(glucose or mannose or allose)-6-phosphate |
| | OCH₃ | 2,3-di-O-methyl-1,5-dideoxy-5-amino-D-(glucose or mannose or allose)-6-phosphate |
| | F or Cl or Br or I | 1,3,5-trideoxy-3-(fluoro or chloro or bromo or iodo)-2-O-methyl-5-amino-D-(glucose or mannose or allose)-6-phosphate |
| NH₂ | OH | 1,5-dideoxy-5-amino-D-(glucosamine or mannosamine or allosamine)-6-phosphate |
| | H | 1,2,3,5-tetradeoxy-2,5-diamino-D-(glucose or mannose or allose) |
| | OCH₃ | 3-O-methyl-1,5-dideoxy-5-amino-D-(glucosamine or mannosamine or allosamine)-6-phosphate |
| | F or Cl or Br or | 1,3,5-trideoxy-3-(fluoro or chloro or bromo or iodo)-5-amino-D-(glucosamine or mannosamine or allosamine)-6-phosphate |
| NHCOCH₃ | OH | 1,2,5-trideoxy-5-amino-2-acetamido-D-(glucose or mannose or allose)-6-phosphate |
| | H | 1,2,3,5-tetradeoxy-2-acetamido-5-amino-D-(glucose or mannose or allose)-6-phosphate |
| | OCH₃ | 3-O-methyl-2-acetamido-1,2,5-trideoxy-5-amino-D-(glucose or mannose or allose)-6-phosphate |
| | F or CI or Br or I | 1,2,3,5-tetradeoxy-3-(fluoro or chloro or bromo or iodo)-2-acetamido-5-amino-D-(glucose or mannose or allose)-6-phosphate |
| NHSO₃H | OH | 1,2,5-trideoxy-2-sulfamino-5-amino-D-(glucose or mannose or allose)-6-phosphate |
| | H | 1,2,3,5-tetraeoxy-2-sulfamino-5-amino-D-(glucose or mannose or allose)-6-phosphate |
| | OCH₃ | 3-O-methyl-1,2,5-trideoxy-2-sulfamino-5-amino-D-(glucose or mannose or allose)-6-phosphate |
| | F or CI or Br or I | 1,2,3,5-tetradeoxy-3-(fluoro or chloro or bromo or iodo)-2-sulfamino-5-amino-D-(glucose or mannose or allose)-6-phosphate |
| F or CI or Br or I | OH | 1,2,5-trideoxy-2-(fluoro or chloro or bromo or iodo)-5-amino-D-(glucose or mannose or allose)-6-phosphate |
| | H | 1,2,3,5-tetradeoxy-2-(fluoro or chloro or bromo or iodo)-5-amino-D-(glucose or mannose or allose)-6-phosphate |
| | OCH₃ | 3-O-methyl-1,2,5-trideoxy-2-(fluoro or chloro or bromo or iodo)-5-aminO-D-(glucose or mannose or allose)-6-phosphate |
| | F or Cl or Br or I | 1,2,3,5-tetradeoxy-2,3-(fluoro or chloro or bromo or iodo)-5-amino-D-(glucose or mannose or allose)-6-phosphate |

### Salts of the C-6 phosphorylated D-aldohexoses

As mentioned before, the C-6 phosphorylated D-aldohexoses and derivatives thereof of the invention also include any inorganic and organic salt of the phosphorylated compounds. Preferably, such salts include pharmaceutically acceptable basic addition salts, in particular pharmaceutically acceptable metal salts, ammonium and alkylated ammonium salts. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine and the like. Such salts include also pharmaceutically acceptable acid addition salts, in particular for the D-aldohexose derivatives containing an amino group (an example is D-glucosamine). Representative examples of suitable inorganic acids include hydrochloric, hydrobromic, hydroiodic, phosphoric, sulfuric, perchloric, and the like. Representative examples of suitable organic acids include formic, acetic, trichloroacetic, trifluoroacetic, propionic, benzoic, cinnamic, citric, fumaric, and the like. Further examples of pharmaceutically acceptable inorganic or organic acid addition salts include the pharmaceutically acceptable salts listed in any review or book dealing with pharmaceutical salts, in particular in Berge et al. (Pharmaceutical salts, J Pharm Sci, 1977, 66: 1-19).

The invention further relates to compounds corresponding to disaccharides, polysaccharides, polymers or any compound derived from the enzymatic or chemical polymerization of one or several phosphorylated D-aldohexose derivatives of the present invention, and uses thereof, including as medicaments.

### Use of as key intermediates for the development of other derivatives

6-C phosphorylated D-aldohexose and D-aldohexose derivatives of the present invention can be directly used as active ingredients as cosmetics or as active substances alone or in combination with other products, including other active compounds with synergistic or complementary activities, or with stabilizers or excipients.

6-C phosphorylated D-aldohexose and D-aldohexose derivatives obtained by the method of the present invention can further be used directly or be considered as intermediate entities for the enzymatic of chemical synthesis of other molecules. In one embodiment, said 6-C phosphorylated D-aldohexose and D-aldohexose derivatives obtained by the method of the present invention are used to synthesize disaccharides or polysaccharides by any convenient technique either chemical or enzymatic process or a combination thereof. Said 6-C phosphorylated D-aldohexose and D-aldohexose derivatives can also be used to create polymers or derivatives thereof including detergents or moisturizers.

As the enzymatic reaction used in the present invention concerns specific C-6 hydroxyl position on the pyranose ring of the D-aldohexose or derivatives thereof, the other hydroxyl groups can for example be used as a reactive group in a chemical reaction to create ester bonds (for instance, when reacted with carboxylic acids activated or not), ether bonds (when reacted with epoxides for instance), sulphate or phosphate bonds. Such modifications can improve already existing properties of the 6-C phosphorylated D-aldohexose and D-aldohexose derivatives of the present invention, or provide new properties for specific applications (higher therapeutic efficiency, lower cytotoxicity, ...).

They can be used in different fields of applications such as dermocosmetics preparations, fine chemistry specialties, food and feed application, detergents compositions, and products for therapeutic or diagnostic purposes.

### Compositions of the invention

The present invention also concerns a composition, preferably a pharmaceutical or cosmetic composition, comprising a phosphorylated aldohexose derivatives of the present invention or a disaccharide, polysaccharide, polymer or any compound obtained from enzymatic or chemical polymerization of one or several said phosphorylated aldohexose derivatives of the present invention. The pharmaceutical or cosmetic composition preferably further comprises a physiologically acceptable carrier or support.

The compositions of the present invention may be administered orally, parenterally, by inhalation spray, topically, rectally, nasally, buccally, vaginally or via reservoir system (e.g. an implant). The term "parenteral" as used herein includes subcutaneous, intravenous, intramuscular, intra-articular, intra-synovial, intrasternal, intrathecal, intrahepatic, intralesional and intracranial injection or infusion techniques. Preferably, the composition of the present invention are administered orally, by inhalation spray, topically, rectally, nasally, buccally, or vaginally. In a preferred embodiment, the pharmaceutical or cosmetic composition is administered topically.
New types of cosmetic products are constantly being developed, and new raw materials are adding to the cosmetic chemist's selection of personal care ingredients. The 6-C phosphorylated D-aldohexose and D-aldohexose derivatives obtained by the method of the present invention can easily be incorporated in a large panel of cosmetic products. Such preparations are well known from the man of the art: it can be creams, sticks, shampoo, shower gels, lotions, soaps, emulsions, gels. These formulations can include other ingredients such as but not limited to: deionized water, magnesium, aluminium silicate, xanthan gum, nylon-12, sodium PCA, propylene glycol, red iron oxides, talc, yellow iron oxides, black iron oxides, titanium dioxide, glyceryl stearate, stearic acid, DEA-cetyl phosphate, methylparaben, butylparaben, ethylparaben, propylparaben, isotearyl neopentanoate, isopropyl palmitate, ethylene/propylene/styrene, copolymers, butylene/ethylene/styrene copolymer, isopropyl palmitate, phenoxyethanol, tocopheryl acetate, glycerin, triethanolamine, stearic acid, propylene glycol stearate, mineral oil, buthylene/ethylene/styrene copolymer, diazolidinyl urea, hydrogenated polyisobutene, octyl palmitate, tridecyl neopentanoate, isostearyl isotearate, isopropylparaben, isobutyparaben, octyldodecyl neopentanoate, tocopheryl acetate, fragrance, octyl methoxycinnamate, benzophenone, octyl salicylate, isopropyl isostearate, propylene glycol isoceteth-3 acetate, or any combinations thereof.

For their use in therapeutic applications, C-6 phosphorylated D-aldohexose and D-aldohexose derivatives obtained by the method of the present invention can be incorporated in different galenic preparations such as pills, tablets, syrups, creams, lotions, gels using for example packing, standardisation, blending / homogenisation, sterile and nonsterile micronization, granulation/compacting, sieving or any combination thereof. Preparations of said C-6 phosphorylated D-aldohexose and D-aldohexose derivatives can include some excipients of the following non exhaustive list: talc, lactose, magnesium stearate, glycerol monostearate, colloidal silicon dioxide, precipitated silicon dioxide, crosslinked polyvinyl pyrrolidone, dibasic calcium phosphate dihydrate, micro crystalline cellulose, corn starch, povidone, sodium carboxy-methyl cellulose, polysorbate 80, lactic acid, carbomer, cethyl alcohol, isopropyl myristate, isopropyl palmitate, glucose, dextrose, triethanolamine, glycerine, fructose, sucrose, polymers, nanostructures.
The compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.
Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.
The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.
For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.
For ophthalmic use, the compositions may be formulated as solutions in isotonic, pH adjusted sterile saline, either with or without a preservative such as benzylalkonium chloride. Alternatively, for ophthalmic uses, the compositions may be formulated in an ointment such as petrolatum.
The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.
Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Any other embodiments and advantages of the present invention will appear from the following examples, that are illustrative of embodiments of the invention and are not meant to limit the scope of the invention as encompassed by the claims.

### EXAMPLES

### EXAMPLE 1: Synthesis of various D-aldohexoses and D-aldohexose derivatives using an enzymatic extract obtained from a Mycobacterium phlei A TCC 354 or DSMZ 43214

### Step 1: production of an extract containing a polyphosphate-glucose phosphotransferase activity

The Mycobacterium phlei strain was obtained from the Deutsche Sammlung von Mikroorganismen und Zellkulturen GmbH or DSMZ (German Collection of Microorganisms and Cell Cultures); its identification number in this collection is 43214. This strain exists also in the American Type Culture Collection under the reference 354. The inoculants used to produce the enzymatic activity were obtained as follows: the lyophilisate provided by DSMZ was first suspended in 1 ml of sterile water; an aliquote of the suspension was spread on a solid Lowenstein-Jensen egg medium (Loweinstein-Jensen Medium slants; Becton, Dickinson and Co.-DIFCO, ref. 220908/220909) at 37°C. After two days of culture, an aliquote of the microbial biomass was suspended in 100 ml of a culture medium containing 3.0 g/L malt extract (Malt extract, SIGMA-ALDRICH, reference 70167), 12.0 g/L glucose, 3.0 g/L yeast extract (Bacto® Yeast Extract; BD-DIFCO; reference 212750) and 5.0 g/L casein enzymatic hydrolysate (Bacto® Tryptone; BD-DIFCO; reference 211705). The cells were grown 2 days at 30°C and then aliquotes of the culture broth were diluted volume/volume with an aqueous solution of glycerol at 40%. Fractions of 1 ml of the resulting mixtures were introduced into low temperature resistant sterile tubes which were finally placed at -18°C and -78°C: these preparations were used to inoculate the culture media devoted to the production of the microbial biomass from which the polyphosphate-glucose phosphotransferase activity will be extracted.
The production of the microbial biomass containing the polyphosphate-glucose phosphotransferase activity was achieved as follows: as a preculture, 20 ml of culture medium prepared as described above (3.0 g/l malt extract, 12.0 g/l glucose, 3.0 g/L yeast extract and 5.0 g/L casein enzymatic hydrolysate) were inculated with 1 ml of previously described glycerol containing suspension; the culture broth was incubated under agitation at 30°C during more than 2 days. The production of biomass was carried out in 2 L Erlenmeyer flasks containing 400 ml of a culture medium with the same composition as that of the preculture inoculated by 8 ml of preculture. The cells were grown at 30°C under agitation during more than 4 days; the biomass accumulated on the wall of the flask was resuspended in the culture medium once a day. At the end of the biomass production, the microbial cells were collected by centrifugation.
The polyphosphate-glucose phosphotransferase activity was extracted by disrupting the bacterial cells with glass beads vigorously agitated. After removing the insoluble material by centrifugation, the soluble fraction constituted the enzymatic extract; this extract could be stored at -18°C without measurable loss of activity at least after two cycles of freezing/defreezing.
The polyphosphate-glucose phosphotransferase activity was measured according to the coupled cofactor enzymatic method proposed by Girbal et al. (Enzyme Microb Technol, 1989 11: 518 - 527). The enzymatic activity in a specific extract was calculated as follows: slope relative to the increase of absorbance at 340 or 365 nm per minute multiplied by the volume of the reaction medium multiplied by the dilution factor of the enzymatic extract divided by the absorbance value of 1 mM (or 1 µmole per ml of reaction medium) of NADPH and divided by the volume of enzymatic preparation introduced into the reaction mixture. One unit of enzymatic activity is the amount of enzyme that produces one micromole of glucose-6-phosphate per minute at 30°C in the assay conditions.

### Step 2: conditions for the synthesis of C-6 phosphorylated D-aldohexoses and D-aldohexose derivatives

The conditions carried out for the synthesis of C-6 phosphorylated D-aldohexose and D-aldohexose derivatives were the followings:
Sodium hexametaphosphate (Sigma-Aldrich, P8510; average polymerization degree: 12-13; molecular weight: 1336,48): 7.5 mM.
Acceptor D-aldohexose or D-aldohexose derivative: 150 mM,
Tris-HCl pH 8.5: 82 mM,
MgCl₂: 80 mM,
Polyphosphate-glucose phosphotransferase activity: 0.26 U/ml,
24 hours at 30°C under mild agitation.

### Step 3: analysis of the reaction media

Immediately after mixing all the reactants of the reaction media in one hand and after 24 hours of incubation in the other hand, an aliquote of the reaction media was withdrawn and diluted volume/volume with HCl 0.42 N. The obtained solutions were analyzed by thin layer chromatography and it was looked for the occurrence of a new product in the solution containing the aliquote withdrawn after 24 hours of incubation by comparison with the substances detected in the solution containing the aliquote withdrawn just immediately after mixing the reactants of the reaction medium.
The analysis system used to detect new C-6 phosphorylated D-aldohexoses and D-aldohexose derivatives was the following:
conditions A: the silica gel plate was from Sigma-Aldrich (reference 60800) and the eluent was: 1-butanol 5, water 3, acetic acid 5;
conditions B: the silica gel plate was from Merck (reference 1.05554) and the eluent was: 1-propanol 5, water 3, acetic acid 2;
detection: vanillin 0.6% in ethanol;
load: 5 µL of 2-fold diluted reaction medium

### Step 4: controls

When the occurrence of a 6-C phosphorylated D-aldohexose or D-aldohexose derivative was suspected, the requirement of the combination enzyme/polyphosphate/acceptor to obtain the said product was checked by analyzing a 24 hours incubated reaction medium with all the reactants of the corresponding reaction medium excepted sodium hexametaphosphate.

### Step 5: results

Table 8 reports the results that demonstrate the synthesis of unexpected 6-C phosphorylated D-aldohexoses or D-aldohexose derivatives by the method of the present invention. Indeed, the possibility of phosphorylating substances other than glucose and glucosamine with the polyphosphate-glucose phosphotransferase produced by Mycobacterium phlei was not known and was surprising because this enzyme was described to be highly substrate specific.

**Table 8: synthesis of 6-C phosphorylated D-aldohexose or D-aldohexose derivatives**

| Acceptor | Reaction Medium | Result (24 hours incubation) | Commentary |
|---|---|---|---|
| D-mannose | Analysis conditions: A | | |
| | Complete | Rf = 0.63 is D-mannose Substance with Rf = 0.44 not visible at T = 0 | A new substance is formed exclusively in the complete reaction |
| | Without PolyP* (control) | Rf = 0.63 is D-mannose No other substance detected | medium. It is D-mannose-6-phosphate. |
| | Analysis conditions: B | | |
| | Complete | Rf = 0.73 is D-mannose Substance with Rf = 0.47 not visible at T = 0 | A new substance is formed exclusively in the complete reaction medium. It is D-mannose-6-phosphate. |
| | Without PolyP* (control) | Rf = 0.73 is D-mannose No other substance detected | |
| D-mannosamine | Analysis conditions: A | | |
| | Complete | Rf = 0.57 is D-mannosamine Substance with Rf = 0.31 not visible at T = 0 | A new substance is formed exclusively in the complete reaction medium. It is D- mannosamine-6-phosphate. |
| | Without PolyP* (control) | Rf = 0.57 is D-mannosamine No other substance detected | |
| N-acetyl-D- glucosamine | Analysis conditions: A | | |
| | Complete | Rf = 0.58 is N-acetyl-D-glucosamine Substance with Rf = 0.24 not visible at T = 0 | A new substance is formed exclusively in the complete reaction medium. It is N-acetyl-D- glucosamine-6-phosphate. |
| | Without PolyP* (control) | Rf = 0.58 is N-acetyl-D-glucosamine No other substance detected | |
| D-glucosamine-2-sulfate | Analysis conditions: A | | |
| | Complete | Rf = 0.46 is D-glucosamine-2-sulfate Substance with Rf = 0.27 not visible at T = 0 | A new substance is formed exclusively in the complete reaction medium. It is D- glucosamine-2-sulfate-6-phosphate. |
| | Without PolyP* (control) | Rf = 0.46 is D-glucosamine-2-sulfate No other substance detected | |
| 1,5-anhydro-D-sorbitol | Analysis conditions: A | | |
| | Complete | Rf = 0.58 is 1,5-anhydro-D-sorbitol Substance with Rf = 0.33 not visible at T = 0 | A new substance is formed exclusively in the complete reaction medium. It is 1,5-anhydro-D-sorbitol-6-phosphate. |
| | Without PolyP* (control) | Rf = 0.58 is 1,5-anhydro-D-sorbitol No other substance detected | |
| 2-deoxy-D-glucose | Analysis conditions: A | | |
| | Complete | Rf = 0.72 is 2-deoxy-D-glucose Substance with Rf = 0.54 not visible at T = 0 | A new substance is formed exclusively in the complete reaction medium. It is 2-deoxy-D- glucose-6-phosphate. In addition, the use of a commercial preparation (Sigma-Aldrich D8875) confirmed the presence of 2-deoxy-D-glucose-6-phosphate. |
| | Without PolyP* (control) | Rf = 0.72 is 2-deoxy-D-glucose No other substance detected | |
| 2-deoxy-2-fluoro-D-glucose | Analysis conditions: A | | |
| | Complete | Rf = 0.75 is 2-deoxy-2-fluoro-D-glucose Substance with Rf = 0.53 not visible at T = 0 | A new substance is formed exclusively in the complete reaction medium. It is 2-deoxy-2-fluoro-D-glucose-6-phosphate. |
| | Without PolyP* (control) | Rf = 0.75 is 2-deoxy-2-fluoro-D-glucose No other substance detected | |
| 3-O-methyl-D-glucose | Analysis conditions: A | | |
| | Complete | Rf = 0.66 is 3-O-methyl-D-glucose Substance with Rf = 0.48 not visible at T = 0 | A new substance is formed exclusively in the complete reaction medium. It is 3-O-methyl-D-glucose-6-phosphate. |
| | Without PolyP* (control) | Rf = 0.66 is 3-O-methyl-D-glucose No other substance detected | |
| 1-thio-β-D-glucose (glucothiose, sodium salt) | Analysis conditions: B | | |
| | Complete | Rf = 0.78 is 1-thio-β-D-glucose Substance with Rf = 0.63 not visible at T = 0 | A new substance is formed exclusively in the complete reaction medium. It is 1-thio-β-D-glucose-6-phosphate. |
| | Without PolyP* (control) | Rf = 0.78 is 1-thio-β-D-glucose No other substance detected | |
| 5-thio-D-glucose | Analysis conditions: B | | |
| | Complete | Rf = 0.81 is 5-thio-D-glucose Substance with Rf = 0.64 not visible at T = 0 | A new substance is formed exclusively in the complete reaction medium. It is 5-thio-D-glucose-6-phosphate. |
| | Without PolyP* (control) | Rf = 0.81 is 5-thio-D-glucose No other substance detected | |
| D-allose | Analysis conditions: B | | |
| | Complete | Rf = 0.75 is D-allose Substance with Rf = 0.63 not visible at T = 0 | A new substance is formed exclusively in the complete reaction medium. It is D-allose-6-phosphate. |
| | Without PolyP* (control) | Rf = 0.75 is D-allose No other substance detected | |

| | | | |
|---|---|---|---|
| PolyP: sodium hexametaphosphate | | | |

### EXAMPLE 2: D-aldohexoses and D-aldohexose derivatives that could not be phosphorylated using an enzymatic extract obtained from a Mycobacterium phlei A TCC 354 or DSMZ 43214

The enzymatic extract obtained in example 1 was used to attempt the synthesis of D-aldohexoses and D-aldohexose derivatives different from those described in example 1. The synthesis conditions as well as the techniques used to detect the phosphorylated substances were the same than those described in exemple 1.
The results are the followings:
D-altrose, characteristic in that the configuration at C-2 is the same than that of D-mannose and that the configuration at C-3 is the same than that of D-allose, is not phosphorylated: no new substance was detected in the 24 hours-incubation medium;
D-galactose, characteristic in that the configurations at C-2 and C-3 are the same than those of D-glucose and that the configuration at C-4 is the opposite than that of D-glucose, is not phosphorylated: no new substance was detected in the 24 hours-incubation medium;
α-D-glucose-1-phosphate, characteristic in that the alcohol generated by hemiacetal ring closure is linked to a phosphate group, is not phosphorylated: no new substance was detected in the 24 hours-incubation medium;
D-fructose, characteristic in that the molecule contains a ketonic carbonyl or potential ketonic carbonyl group (ketose) and does not contain an aldehydic carbonyl or potential aldehydic carbonyl group, is not phosphorylated: no new substance was detected in the 24 hours-incubation medium;
D-ribose, characteristic in that the monosaccharide is a D-aldopentose, is not phosphorylated: no new substance was detected in the 24 hours-incubation medium.

### EXAMPLE 3: C-6 phosphorylated D-aldohexoses and D-aldohexose derivatives synthesis with enzymatic extracts different from that obtained from a Mycobacterium phlei A TCC 354 or DSMZ 43214

*The bacterial strains used are the followings:*
Mycobacterium phlei ATCC 11758,
Mycobacterium smegmatis DSMZ 43465 (equivalent to ATCC 607),
Mycobacterium smegmatis ATCC 607 (equivalent to DSMZ 43465)
Mycobacterium smegmatis ATCC 700084 (or mc² 155)

### Step 1: production of extracts containing a polyphosphate-glucose phosphotransferase activity

The inoculants used to produce the enzymatic activity were obtained as previously described for the Mycobacterium phlei DSMZ 43214 strain, excepted that the composition of the culture medium was that described by Middlebrook (Middlebrook 7H9 broth base, Sigma-Aldrich M-0178 at 2.35 g/450 ml) complemented by glycerol at the concentration of 20 ml/L. The cells were grown at 37°C in agitated Erlenmeyer flasks.
The production of the microbial biomass containing the polyphosphate-glucose phosphotransferase activity was achieved as previously described, excepted that the composition of the culture medium was that described by Middlebrook (Middlebrook 7H9 broth base, Sigma-Aldrich M-0178 at 2.35 g/450 ml) complemented by glycerol at the concentration of 20 ml/L. The cells were grown at 37°C in agitated Erlenmeyer flasks.
The extraction of the enzymatic activity as well as its measure were carried out as previously described.

### Step 2: conditions for the synthesis of C-6 phosphorylated D-aldohexoses and D-aldohexose derivatives

The conditions carried out for the synthesis of C-6 phosphorylated D-aldohexose and D-aldohexose derivatives were the followings:
Sodium hexametaphosphate (Sigma-Aldrich, P8510): 7.5 mM.
Acceptor D-aldohexose or D-aldohexose derivative: 150 mM,
Tris-HCl pH 8.5: 82 mM,
MgCl₂: 80 mM,
*Polyphosphate-glucose phosphotransferase activity:*
Mycobacterium phlei ATCC 11758: *0.026 U*/*ml,*
Mycobacterium smegmatis DSMZ 43465: *0.020 U*/*ml,*
Mycobacterium smegmatis ATCC 607: *0.021 U*/*ml,*
Mycobacterium smegmatis ATCC 700084 (or mc² 155): *0.025 U*/*ml,*
24 hours at 30°C under mild agitation.

### Step 3: analysis of the reaction media

The analysis of the reaction media was carried out as previously described.

### Step 4: controls

When the occurrence of a 6-C phosphorylated D-aldohexose or D-aldohexose derivative was suspected, the requirement of the combination enzyme/polyphosphate/acceptor to obtain the said product was checked by analyzing a 24 hours incubated reaction medium with all the reactants of the corresponding reaction medium excepted sodium hexametaphosphate.

### Step 5: results

Table 9 reports that all the enzymatic preparations were able to catalyze the synthesis of D-glucothiose-6-phosphate and 2-deoxy-D-glucose-6-phosphate. As it was observed for the Mycobacterium phlei DSMZ 43214 strain, D-glucose-1,6-bisphosphate could not be synthesized.

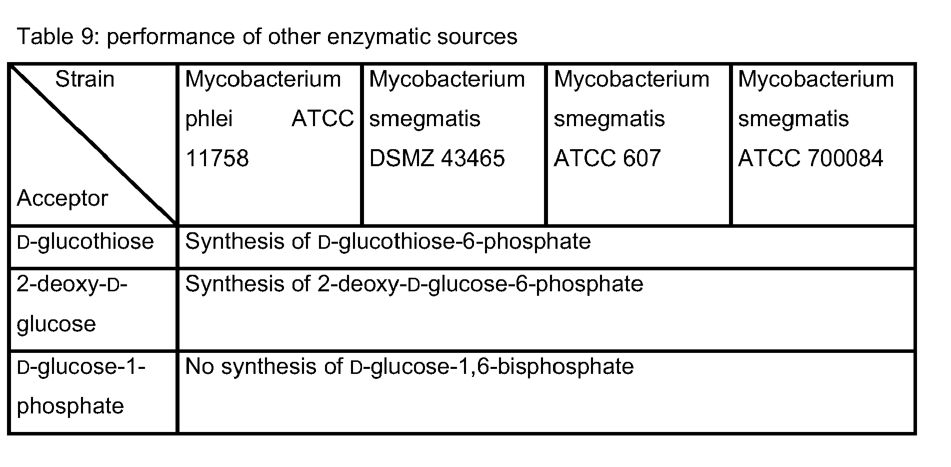

## Claims

1. A method for preparing a C-6 phosphorylated D-aldohexose and a C-6 phosphorylated D-aldohexose derivative, comprising reacting polyphosphate, as sole source of phosphate donor, and a polyphosphate-glucose phosphotransferase in an aqueous solution with a D-aldohexose or a D-aldohexose derivative having the general formula (I): 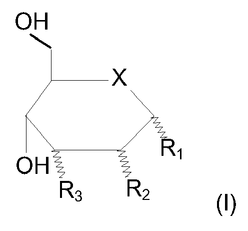
R₁ is selected from the group consisting of OH, SH, and H;
X is selected from the group consisting of O, NH, and S;
R₂ and R₃ are positioned as follows:
both below or above, or R₂ is below and R₃ is above,
R₂ represents H, OH, OCH₃, NH₂, NHCOCH₃, NHSO₃H group or an halogen atom, and
R₃ represents H, OH, OCH₃ group or an halogen atom,
with the following provisos:
when R₁ is OH, X is an oxygen atom, R₂ and R₃ are both above, then R₂ and R₃ are not both OH; and
when R₁ is OH, X is an oxygen atom, R₂ is below and R₃ is above and represents OH, then R₂ is not OH or NH₂ group;
or any salt thereof.

2. Method according claim 1, wherein the polyphosphate-glucose phosphotransferase (EC 2.7.1.63) is derived from a species selected from the group consisting of Arthrobacter sp., Corynebacterium sp., Microlunatus sp., Mycobacterium sp., Nocardia sp., Propionibacterium sp., Streptomyces sp., Rhodococus sp. and Bifidobacterium sp..

3. Method according claim 1, wherein the polyphosphate-glucose phosphotransferase derives from a Mycobacterium species, and more preferably from Mycobacterium sp. selected from the group consisting of Mycobacterium tuberculosis (H₃₇Rv), Mycobacterium smegmatis (mc²155), Mycobacterium phlei, Mycobacterium bovis, Mycobacterium avium paratuberculosis, Mycobacterium flavescens, Mycobacterium leprae, Mycobacterium sp. JLS, Mycobacterium sp. KMS, Mycobacterium vanbaalenii, Mycobacterium aurum, Mycobacterium gordonae, Mycobacterium jucho, Mycobacterium pellegrino, or Mycobacterium thermorestibile.

4. Method according to any one of claims 1-3, wherein the D-aldohexose or a D-aldohexose derivative has the general formula (I) wherein R₂ and R₃ are both below.

5. Method according to any one of claims 1-3, wherein the D-aldohexose or a D-aldohexose derivative has the general formula (I) wherein R₂ and R₃ are both above.

6. Method according to any one of claims 1-3, wherein the D-aldohexose or a D-aldohexose derivative has the general formula (I) wherein R₂ is below and R₃ is above.

7. Method according to any one of claims 1-6, wherein the D-aldohexose or a D-aldohexose derivative has the general formula (I) wherein halogen atom is F or Cl.

8. Method according to any one of claims 1-7, wherein the D-aldohexoses or derivatives thereof are selected in the group consisting of:
D-allose
3-deoxy-D-(glucose or mannose or allose)
3-O-methyl-D-(glucose or mannose or allose)
3-deoxy-3-(fluoro or chloro or bromo or iodo)-D-(glucose or mannose or allose) 2-deoxy-D-(mannose or allose)
2,3-dideoxy-D-(mannose or allose)
3-O-methyl-2-deoxy-D-(glucose or mannose or allose)
3-deoxy-3-(fluoro or chloro or bromo or iodo)-2-deoxy-D-(glucose or mannose or allose) 2-O-methyl-D-(glucose or mannose or allose)
3-deoxy-2-O-methyl-D-(glucose or mannose or allose)
2,3-di-O-methyl-D-(glucose or mannose or allose)
3-deoxy-3-(fluoro or chloro or bromo or iodo)-2-O-methyl-D-(glucose or mannose or allose)
D-(mannosamine or allosamine)
2,3-dideoxy-2-aminO-D-(glucose or mannose or allose)
3-O-methyl-D-(glucosamine or mannosamine or allosamine)
3-deoxy-3-(fluoro or chloro or bromo or iodo)-D-(glucosamine or mannosamine or allosamine)
N-acetyl-D-(glucosamine or mannosamine or allosamine)
2,3-dideoxy-2-acetamido-D-(glucose or mannose or allose)
3-O-methyl-N-acetyl-D-(glucosamine or mannosamine or allosamine)
3-deoxy-3-(fluoro or chloro or bromo or iodo)-N-acetyl-D-(glucosamine or mannosamine or allosamine)
2-deoxy-2-sulfamino-D-(glucose or mannose or allose)
2,3-dideoxy-2-sulfamino-D-(glucose or mannose or allose)
3-O-methyl-2-deoxy-2-sulfamino-D-(glucose or mannose or allose)
2,3-dideoxy-3-(fluoro or chloro or bromo or iodo)-2-sulfamino-D-(glucose or mannose or allose)
2-deoxy-2-(fluoro or chloro or bromo or iodo)-D-(glucose or mannose or allose)
2,3-dideoxy-2-(fluoro or chloro or bromo or iodo)-D-(glucose or mannose or allose)
3-O-methyl-2-deoxy-2-(fluoro or chloro or bromo or iodo)-D-(glucose or mannose or allose)
2,3-dideoxy-2,3-(fluoro or chloro or bromo or iodo)-D-(glucose or mannose or allose) 1,5-anhydro-D-(glucitol or mannitol or allitol)
3-deoxy-1,5-anhydro-D-(glucitol or mannitol or allitol)
3-O-methyl-1,5-anhydro-D-(glucitol or mannitol or allitol)
and salts thereof.

9. Method according to anyone of the preceding claims, wherein the D-aldohexoses or derivatives thereof of formula (I) are selected in the group consisting of D-allose, 2-deoxy-D-glucose, 2-deoxy-D-mannose, 2-deoxy-D-allose, 2-O-methyl-D-glucose, 2-O-methyl-D-mannose, 2-O-methyl-D-allose, 2-fluoro-2-deoxy-D-glucose, 2-fluoro-2-deoxy-D-mannose, 2-fluoro-2-deoxy-D-allose, D-mannosamine, D-allosamine, N-acetyl-D-glucosamine, N-acetyl-D-mannosamine, N-acetyl-D-allosamine, 3-O-methyl-D-glucose, 3-O-methyl-D-mannose, 3-O-methyl-D-allose, 1,5 anhydro-D-glucitol, 1,5 anhydro-D-mannitol, 1,5 anhydro-D-allitol, 1-thio-D-glucose, 1-thio-D-mannose, 1-thio-D-allose, 5-thio-D-glucose, 5-thio-D-mannose, 5-thio-D-allose, 5-amino-D-glucose, 5-amino-D-mannose, 5-amino-D-allose, 2-deoxy-2-sulfamino-D-glucose and salts thereof.

10. Method according to anyone of the preceding claims, wherein polyphosphate presents a polymerization degree ranging from 8 to 20.

11. A C-6 phosphorylated D-aldohexose or C-6 phosphorylated D-aldohexose derivative selected in the group consisting of:
3-deoxy-D-(glucose or mannose or allose)-6-phosphate
3-O-methyl-D-(allose)-6-phosphate
3-deoxy-3-chloro-D-(glucose or mannose or allose)-6-phosphate
3-deoxy-3-fluoro-D-(mannose or allose)-6-phosphate
3-deoxy-3-bromo-D-(glucose or mannose or allose)-6-phosphate
3-deoxy-3-iodo-D-(glucose or mannose or allose)-6-phosphate
2-deoxy-D-(mannose or allose)-6-phosphate
2,3-dideoxy-D-(glucose or mannose or allose)-6-phosphate
3-O-methyl-2-deoxy-D-(glucose or mannose or allose)-6-phosphate
3-deoxy-3-(fluoro or chloro or bromo or iodo)-2-deoxy-D-(glucose or mannose or allose)-6-phosphate
2-O-methyl-D-allose-6-phosphate
3-deoxy-2-O-methyl-D-(glucose or mannose or allose)-6-phosphate
2,3-di-O-methyl-D-(glucose or mannose or allose)-6-phosphate
3-deoxy-3-(fluoro or chloro or bromo or iodo)-2-O-methyl-D-(glucose or mannose or allose)-6-phosphate
D-allosamine-6-phosphate
2,3-dideoxy-2-amino-D-(glucose or mannose or allose)-6-phosphate
3-O-methyl-D-(glucosamine or mannosamine or allosamine)-6-phosphate
3-deoxy-3-(fluoro or chloro or bromo or iodo)-D-(glucosamine or mannosamine or allosamine)-6-phosphate
N-acetyl-D-allosamine-6-phosphate
2,3-dideoxy-2-acetamido-D-(glucose or mannose or allose)-6-phosphate
3-O-methyl-N-acetyl-D-(glucosamine or mannosamine or allosamine)-6-phosphate
3-deoxy-3-(fluoro or chloro or bromo or iodo)-N-acetyl-D-(glucosamine or mannosamine or allosamine)-6-phosphate
2-deoxy-2-sulfamino-D-(glucose or mannose or allose)-6-phosphate
2,3-dideoxy-2-sulfamino-D-(glucose or mannose or allose)-6-phosphate
3-O-methyl-2-deoxy-2-sulfamino-D-(glucose or mannose or allose)-6-phosphate
2,3-dideoxy-3-(fluoro or chloro or bromo or iodo)-2-sulfamino-D-(glucose or mannose or allose)-6-phosphate
2-deoxy-2-(fluoro or chloro or bromo or iodo)-D-allose-6-phosphate
2,3-dideoxy-2-(fluoro or chloro or bromo or iodO)-D-(glucose or mannose or allose)-6-phosphate
3-O-methyl-2-deoxy-2-(fluoro or chloro or bromo or iodO)-D-(glucose or mannose or allose)-6-phosphate
2,3-dideoxy-2,3-(fluoro or chloro or bromo or iodo)-D-(glucose or mannose or allose)-6-phosphate
1,5-anhydro-D-(mannitol or allitol)-6-phosphate
3-deoxy-1,5-anhydro-D-(glucitol or mannitol or allitol)-6-phosphate
3-O-methyl-1,5-anhydro-D-(glucitol or mannitol or allitol)-6-phosphate
3-deoxy-3-(fluoro or chloro or bromo or iodo)-1,5-anhydro-D-(glucitol or mannitol or allitol)-6-phosphate
2-deoxy-1,5-anhydro-D-(glucitol or mannitol or allitol)-6-phosphate
2,3-dideoxy-1,5-anhydro-D-(glucitol or mannitol or allitol)-6-phosphate
3-O-methyl-2-deoxy-1,5-anhydro-D-(glucitol or mannitol or allitol)-6-phosphate
2,3-deoxy-3-(fluoro or chloro or bromo or iodo)-1,5-anhydro-D-(glucitol or mannitol or allitol)-6-phosphate
2-O-methyl-1,5-anhydro-D-(glucitol or mannitol or allitol)-6-phosphate
3-deoxy-2-O-methyl-1,5-anhydro-D-(glucitol or mannitol or allitol)-6-phosphate
2,3-di-O-methyl-1,5-anhydro-D-(glucitol or mannitol or allitol)-6-phosphate
3-deoxy-3-(fluoro or chloro or bromo or iodo)-2-O-methyl-1,5-anhydro-D-(glucitol or mannitol or allitol)-6-phosphate
2-deoxy-2-amino-1,5-anhydro-D-(glucitol or mannitol or allitol)-6-phosphate
2,3-dideoxy-2-amino-1,5-anhydro-D-(glucitol or mannitol or allitol)-6-phosphate
3-O-methyl-2-deoxy-2-amino-1,5-anhydro-D-(glucitol or mannitol or allitol)-6-phosphate
2,3-dideoxy-3-(fluoro or chloro or bromo or iodo)-2-amino-1,5-anhydro-D-(glucitol or mannitol or allitol)-6-phosphate
2-deoxy-2-acetamido-1,5-anhydro-D-(glucitol or mannitol or allitol)-6-phosphate
2,3-dideoxy-2-acetamido-1,5-anhydro-D-(glucitol or mannitol or allitol)-6-phosphate
3-O-methyl-2-deoxy-2-acetamido-1,5-anhydro-D-(glucitol or mannitol or allitol)-6-phosphate
2,3-dideoxy-3-(fluoro or chloro or bromo or iodo)-2-acetamido-1,5-anhydro-D-(glucitol or mannitol or allitol)-6-phosphate
2-deoxy-2-sulfamino-1,5-anhydro-D-(glucitol or mannitol or allitol)-6-phosphate
2,3-dideoxy-2-sulfamino-1,5-anhydro-D-(glucitol or mannitol or allitol)-6-phosphate
3-O-methyl-2-deoxy-2-sulfamino-1,5-anhydro-D-(glucitol or mannitol or allitol)-6-phosphate
2,3-dideoxy-3-(fluoro or chloro or bromo or iodo)-2-sulfamino-1,5-anhydro-D-(glucitol or mannitol or allitol)-6-phosphate
2-deoxy-2-(fluoro or chloro or bromo or iodo)-1,5-anhydro-D-(glucitol or mannitol or allitol)-6-phosphate
2,3-dideoxy-2-(fluoro or chloro or bromo or iodo)-1,5-anhydro-D-(glucitol or mannitol or allitol)-6-phosphate
3-O-methyl-2-deoxy-2-(fluoro or chloro or bromo or iodo)-1,5-anhydro-D-(glucitol or mannitol or allitol)-6-phosphate
2,3-dideoxy-2,3-(fluoro or chloro or bromo or iodo)-1,5-anhydro-D-(glucitol or mannitol or allitol)-6-phosphate
D-glucothiose-6-phosphate or D-mannothiose-6-phosphate or D-allothiose-6-phosphate 3-deoxy-D-(glucothiose or mannothiose or allothiose)-6-phosphate
3-O-methyl-D-(glucothiose or mannothiose or allothiose)-6-phosphate
3-deoxy-3-(fluoro or chloro or bromo or iodo)-D-(glucothiose or mannothiose or allothiose)-6-phosphate
2-deoxy-D-(glucothiose or mannothiose or allothiose)-6-phosphate
2,3-dideoxy-D-(glucothiose or mannothiose or allothiose)-6-phosphate
3-O-methyl-2-deoxy-D-(glucothiose or mannothiose or allothiose)-6-phosphate
3-deoxy-3-(fluoro or chloro or bromo or iodo)-2-deoxy-D-(glucothiose or mannothiose or allothiose)-6-phosphate
2-O-methyl-D-(glucothiose or mannothiose or allothiose)-6-phosphate
3-deoxy-2-O-methyl-D-(glucothiose or mannothiose or allothiose)-6-phosphate 2,3-di-O-methyl-D-(glucothiose or mannothiose or allothiose)-6-phosphate
3-deoxy-3-(fluoro or chloro or bromo or iodo)-2-O-methyl-D-(glucothiose or mannothiose or allothiose)-6-phosphate
1-thio-D-(glucosamine or mannosamine or allosamine)-6-phosphate
2,3-dideoxy-2-amino-D-(glucothiose or mannothiose or allothiose)-6-phosphate
3-O-methyl-1-thio-D-(glucosamine or mannosamine or allosamine)-6-phosphate
3-deoxy-3-(fluoro or chloro or bromo or iodo)-1-thio-D-(glucosamine or mannosamine or allosamine)-6-phosphate
N-acetyl-1-thio-o-(glucosamine or mannosamine or allosamine)-6-phosphate
2,3-dideoxy-2-acetamido-D-(glucothiose or mannothiose or allothiose)-6-phosphate
3-O-methyl-N-acetyl-1-thio-D-(glucosamine or mannosamine or allosamine)-6-phosphate
3-deoxy-3-(fluoro or chloro or bromo or iodo)-N-acetyl-1-thio-D-(glucosamine or mannosamine or allosamine)-6-phosphate
2-deoxy-2-sulfaminO-D-(glucothiose or mannothiose or allothiose)-6-phosphate
2,3-dideoxy-2-sulfamino-D-(glucothiose or mannothiose or allothiose)-6-phosphate
3-O-methyl-2-deoxy-2-sulfamino-D-(glucothiose or mannothiose or allothiose)-6-phosphate
2,3-dideoxy-3-(fluoro or chloro or bromo or iodo)-2-sulfamino-D-(glucothiose or mannothiose or allothiose)-6-phosphate
2-deoxy-2-(fluoro or chloro or bromo or iodo)-D-(glucothiose or mannothiose or allothiose)-6-phosphate
2,3-dideoxy-2-(fluoro or chloro or bromo or iodo)-D-(glucothiose or mannothiose or allothiose)-6-phosphate
3-O-methyl-2-deoxy-2-(fluoro or chloro or bromo or iodo)-D-(glucothiose or mannothiose or allothiose)-6-phosphate
2,3-dideoxy-2,3-(fluoro or chloro or bromo or iodo)-D-(glucothiose or mannothiose or allothiose)-6-phosphate
5-thio-D-mannose-6-phosphate or 5-thio-D-allose-6-phosphate
3-deoxy-5-thio-D-(glucose or mannose or allose)-6-phosphate
3-O-methyl-5-thio-D-(glucose or mannose or allose)-6-phosphate
3-deoxy-3-(fluoro or chloro or bromo or iodo)-5-thio-D-(glucose or mannose or allose)-6-phosphate
2-deoxy-5-thio-D-(glucose or mannose or allose)-6-phosphate
2,3-dideoxy-5-thio-D-(glucose or mannose or allose)-6-phosphate
3-O-methyl-2-deoxy-5-thio-D-(glucose or mannose or allose)-6-phosphate
2,3-dideoxy-3-(fluoro or chloro or bromo or iodo)-5-thiO-D-(glucose or mannose or allose)-6-phosphate
2-O-methyl-5-thio-D-(glucose or mannose or allose)-6-phosphate
3-deoxy-2-O-methyl-5-thio-D-(glucose or mannose or allose)-6-phosphate
2,3-di-O-methyl-5-thio-D-(glucose or mannose or allose)-6-phosphate
3-deoxy-3-(fluoro or chloro or bromo or iodo)-2-O-methyl-5-thio-D-(glucose or mannose or allose)-6-phosphate
5-thio-D-(glucosamine or mannosamine or allosamine)-6-phosphate
2,3-dideoxy-2-amino-5-thio-D-(glucose or mannose or allose)-6-phosphate
3-O-methyl-5-thio-D-(glucosamine or mannosamine or allosamine)-6-phosphate
3-deoxy-3-(fluoro or chloro or bromo or iodo)-5-thio-D-(glucosamine or mannosamine or allosamine)-6-phosphate
N-acetyl-5-thio-D-(glucosamine or mannosamine or allosamine)-6-phosphate
2,3-dideoxy-2-acetamido-5-thio-D-(glucose or mannose or allose)-6-phosphate
3-O-methyl-N-acetyl-5-thio-D-(glucosamine or mannosamine or allosamine)-6-phosphate
3-deoxy-3-(fluoro or chloro or bromo or iodo)-N-acetyl-5-thio-D-(glucosamine or mannosamine or allosamine)-6-phosphate
2-deoxy-2-sulfamino-5-thio-D-(glucose or mannose or allose)-6-phosphate
2,3-dideoxy-2-sulfamino-5-thio-D-(glucose or mannose or allose)-6-phosphate
3-O-methyl-2-deoxy-2-sulfamino-5-thio-D-(glucose or mannose or allose)-6-phosphate
2,3-dideoxy-3-(fluoro or chloro or bromo or iodo)-2-sulfamino-5-thio-D-(glucose or mannose or allose)-6-phosphate
2-deoxy-2-(fluoro or chloro or bromo or iodo)-5-thio-D-(glucose or mannose or allose)-6-phosphate
2,3-dideoxy-2-(fluoro or chloro or bromo or iodo)-5-thio-D-(glucose or mannose or allose)-6-phosphate
3-O-methyl-2-deoxy-2-(fluoro or chloro or bromo or iodo)-5-thio-D-(glucose or mannose or allose)-6-phosphate
2,3-dideoxy-2,3-(fluoro or chloro or bromo or iodo)-5-thio-D-(glucose or mannose or allose)-6-phosphate
5-amino-D-allose-6-phosphate
3,5-dideoxy-5-amino-D-(glucose or mannose or allose)-6-phosphate 3-O-methyl-5-deoxy-5-amino-D-(glucose or mannose or allose)-6-phosphate
3,5-dideoxy-3-(fluoro or chloro or bromo or iodo)-5-amino-D-(glucose or mannose or allose)-6-phosphate
2,5-dideoxy-5-aminO-D-(glucose or mannose or allose)-6-phosphate
2,3,5-trideoxy-5-aminO-D-(glucose or mannose or allose)-6-phosphate
3-O-methyl-2,5-dideoxy-5-amino-D-(glucose or mannose or allose)-6-phosphate
2,3,5-trideoxy-3-(fluoro or chloro or bromo or iodo)-5-aminO-D-glucose or mannose or allose)-6-phosphate
2-O-methyl-5-deoxy-5-amino-D-(glucose or mannose or allose)-6-phosphate
3,5-dideoxy-2-O-methyl-5-amino-D-(glucose or mannose or allose)-6-phosphate
2,3-di-O-methyl-5-deoxy-5-amino-D-(glucose or mannose or allose)-6-phosphate
3,5-dideoxy-3-(fluoro or chloro or bromo or iodo)-2-O-methyl-5-amino-D-(glucose or mannose or allose)-6-phosphate
5-deoxy-5-amino-D-(glucosamine or mannosamine or allosamine)-6-phosphate
2,3,5-trideoxy-2,5-diamino-D-(glucose or mannose or allose)-6-phosphate 3-O-methyl-5-deoxy-5-amino-D-(glucosamine or mannosamine or allosamine)-6-phosphate
3,5-dideoxy-3-(fluoro or chloro or bromo or iodo)-5-amino-D-(glucosamine or mannosamine or allosamine)-6-phosphate
2,5-dideoxy-5-amino-2-acetamido-D-(glucose or mannose or allose)-6-phosphate
2,3,5-trideoxy-2-acetamido-5-amino-D-(glucose or mannose or allose)-6-phosphate 3-O-methyl-2-acetamido-2,5-dideoxy-5-amino-D-(glucose or mannose or allose)-6-phosphate
2,3,5-trideoxy-3-(fluoro or chloro or bromo or iodo)-2-acetamido-5-amino-D-(glucose or mannose or allose)-6-phosphate
2,5-dideoxy-2-sulfamino-5-amino-D-(glucose or mannose or allose)-6-phosphate
2,3,5-trideoxy-2-sulfamino-5-amino-D-(glucose or mannose or allose)-6-phosphate
3-O-methyl-2,5-dideoxy-2-sulfamino-5-amino-D-(glucose or mannose or allose)-6-phosphate
2,3,5-trideoxy-3-(fluoro or chloro or bromo or iodo)-2-sulfamino-5-amino-D-(glucose or mannose or allose)-6-phosphate
2,5-dideoxy-2-(fluoro or chloro or bromo or iodo)-5-amino-D-(glucose or mannose or allose)-6-phosphate
2,3,5-trideoxy-2-(fluoro or chloro or bromo or iodo)-5-amino-D-(glucose or mannose or allose)-6-phosphate
3-O-methyl-2,5-dideoxy-2-(fluoro or chloro or bromo or iodo)-5-amino-D-(glucose or mannose or allose)-6-phosphate
2,3,5-trideoxy-2,3-(fluoro or chloro or bromo or iodo)-5-amino-D-(glucose or mannose or allose)-6-phosphate
1,5-dideoxy-5-amino-D-(glucose or mannose or allose)-6-phosphate
1,3,5-trideoxy-5-amino-D-(glucose or mannose or allose)-6-phosphate
3-O-methyl-1,5-dideoxy-5-amino-D-(glucose or mannose or allose)-6-phosphate 1,3,5-trideoxy-3-(fluoro or chloro or bromo or iodo)-5-amino-D-(glucose or mannose or allose)-6-phosphate
1,2,5-trideoxy-5-amino-D-(glucose or mannose or allose)-6-phosphate
1,2,3,5-tetradeoxy-5-amino-D-(glucose or mannose or allose)-6-phosphate
3-O-methyl-1,2,5-trideoxy-5-amino-D-(glucose or mannose or allose)-6-phosphate
1,2,3,5-tetradeoxy-3-(fluoro or chloro or bromo or iodo)-5-amino-D-glucose or mannose or allose)-6-phosphate
2-O-methyl-1,5-dideoxy-5-amino-D-(glucose or mannose or allose)-6-phosphate 1,3,5-trideoxy-2-O-methyl-5-amino-D-(glucose or mannose or allose)-6-phosphate
2,3-di-O-methyl-1 ,5-dideoxy-5-amino-D-(glucose or mannose or allose)-6-phosphate
1,3,5-trideoxy-3-(fluoro or chloro or bromo or iodo)-2-O-methyl-5-amino-D-(glucose or mannose or allose)-6-phosphate
1,5-dideoxy-5-amino-D-(glucosamine or mannosamine or allosamine)-6-phosphate
1,2,3,5-tetradeoxy-2,5-diamino-D-(glucose or mannose or allose) 3-O-methyl-1,5-dideoxy-5-amino-D-(glucosamine or mannosamine or allosamine)-6-phosphate
1,3,5-trideoxy-3-(fluoro or chloro or bromo or iodo)-5-amino-D-(glucosamine or mannosamine or allosamine)-6-phosphate
1,2,5-trideoxy-5-amino-2-acetamido-D-(glucose or mannose or allose)-6-phosphate 1,2,3,5-tetradeoxy-2-acetamido-5-amino-D-(glucose or mannose or allose)-6-phosphate
3-O-methyl-2-acetamido-1,2,5-trideoxy-5-amino-D-(glucose or mannose or allose)-6-phosphate
1,2,3,5-tetradeoxy-3-(fluoro or chloro or bromo or iodo)-2-acetamido-5-amino-D-(glucose or mannose or allose)-6-phosphate
1,2,5-trideoxy-2-sulfamino-5-amino-D-(glucose or mannose or allose)-6-phosphate
1,2,3,5-tetraeoxy-2-sulfamino-5-amino-D-(glucose or mannose or allose)-6-phosphate 3-O-methyl-1,2,5-trideoxy-2-sulfamino-5-amino-D-(glucose or mannose or allose)-6-phosphate
1,2,3,5-tetradeoxy-3-(fluoro or chloro or bromo or iodo)-2-sulfamino-5-amino-D-(glucose or mannose or allose)-6-phosphate
1,2,5-trideoxy-2-(fluoro or chloro or bromo or iodo)-5-amino-D-(glucose or mannose or allose)-6-phosphate
1,2,3,5-tetradeoxy-2-(fluoro or chloro or bromo or iodo)-5-aminO-D-(glucose or mannose or allose)-6-phosphate
3-O-methyl-1,2,5-trideoxy-2-(fluoro or chloro or bromo or iodo)-5-amino-D-(glucose or mannose or allose)-6-phosphate
1,2,3,5-tetradeoxy-2,3-(fluoro or chloro or bromo or iodo)-5-amino-D-(glucose or mannose or allose)-6-phosphate
and salts thereof.

12. Disaccharides, polysaccharides, polymers or compounds derived from the enzymatic or chemical polymerization of one or several phosphorylated D-aldohexose derivatives as defined in the preceding claim, or salts thereof.

13. Composition comprising a phosphorylated aldohexose derivatives as defined in claim 11 or a disaccharide, polysaccharide, polymer or any compound obtained from enzymatic or chemical polymerization of one or several said phosphorylated aldohexose derivatives as defined in claim 11.
